(19) Europäisches Patentamt / European Patent Office / Office européen des brevets

(11) **EP 2 815 701 A1**

(12) **EUROPEAN PATENT APPLICATION**
published in accordance with Art. 153(4) EPC

(43) Date of publication:
**24.12.2014 Bulletin 2014/52**

(51) Int Cl.:
***A61B 8/00*** *(2006.01)*

(21) Application number: **13749172.6**

(22) Date of filing: **23.01.2013**

(86) International application number:
**PCT/JP2013/051305**

(87) International publication number:
**WO 2013/121842 (22.08.2013 Gazette 2013/34)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **15.02.2012 JP 2012030991**

(71) Applicant: **Hitachi, Ltd.**
**Tokyo 100-8280 (JP)**

(72) Inventors:
• **IKEDA, Teiichiro**
**Chiyoda-ku, Tokyo 100-8280 (JP)**

• **MASUZAWA, Hiroshi**
**Chiyoda-ku, Tokyo 100-8280 (JP)**
• **TABARU, Marie**
**Chiyoda-ku, Tokyo 100-8280 (JP)**
• **TAKANO, Shinta**
**Chiyoda-ku, Tokyo 100-8280 (JP)**
• **HASHIBA, Kunio**
**Chiyoda-ku, Tokyo 100-8280 (JP)**

(74) Representative: **Strehl Schübel-Hopf & Partner**
**Maximilianstrasse 54**
**80538 München (DE)**

(54) **ULTRASONIC IMAGING DEVICE**

(57) According to a method of adaptive signal processing, compensation for deterioration of image quality caused by wavefront distortion is provided in an ultrasound imaging apparatus, with a low computing amount and with a precision. The elements 106 receive ultrasound signals from a test subject, and the delay circuit 204 delays each of the signals received by the plural elements in association with a predetermined position of a receive focus. The peripheral information operator 205 acquires from the post-delay received signals, information items as to plural points on the receive focus and in the peripheral region thereof, respectively. By way of example, the peripheral information operator obtains adaptive weights in association with plural steering angles. The peripheral information combiner 206 combines the plural information items (adaptive weights), and uses thus combined information to generate a beamforming output. Alternatively, the beamforming outputs are generated by using the plural adaptive weights and received signals, and thereafter those outputs are combined.

FIG.2

**Description**

**Technical Field**

The present invention relates to an ultrasound imaging technique for imaging the inside of a test subject, through the use of ultrasound waves.

**Background Art**

Ultrasound imaging is a technique for non-invasively creating an image of the inside of a test subject such as a human body, through the use of ultrasound waves (being a sound wave not intended for hearing, and generally a high-frequency sound wave having 20 kHz or higher). By way of example, a medical ultrasound imaging apparatus will be briefly explained. An ultrasound probe transmits the ultrasound waves to the inside of a patient body, and receives echo signals reflected from the inside of the patient. The received signals are subjected to signal processing in one or both of the ultrasound probe and the main unit of the ultrasound imaging apparatus, and thereafter transferred to a monitor, and then, an ultrasound image is displayed thereon. More specifically, for example, a transmit beamformer in the main unit of the ultrasound imaging apparatus generates signals of a transmission beam, allowing the signals to pass through the transmit-receive separation circuit (T/R), and thereafter transfers the signals to the ultrasound probe. The ultrasound probe sends out ultrasound waves. After receiving the echo signals from the internal body, the ultrasound probe transmits the signals to the main unit of the imaging apparatus. In the main unit of the imaging apparatus, the received signals pass through the transmit-receive separation circuit again, being subjected to beamforming process in the receive beamformer, and then those signals are transmitted to an image processor. The image processor executes various image processing through the use of various filters, a scan converter, and the like. Finally, the monitor displays an ultrasound image.

As described above, a general ultrasound diagnostic apparatus is made up of three techniques; transmit beamforming, receive beamforming, and a backend image processing. Particularly, since the beamformers for transmitting and receiving perform signal processing at an RF (high-frequency) level, algorithms and implementation architecture in the beamformers decide a basic image quality of the ultrasound image. Therefore, the beamformers serve as major parts of the apparatus.

The receive beamformer assigns a delay time to each received signal (received data) in plural elements that constitute the ultrasound probe, the delay time distributing an amount of delay in a concave form, in association with the relations between a focal position and the element positions, and after virtually achieving focus (focused) at a certain point in space, the received signal data items are summed up. This method is referred to as a beamforming according to a delay-and-sum method. In this delay-and-sum method, the received data items that are received by the plural elements in the ultrasound diagnostic apparatus are multiplied by a fixed weight vector stored in the diagnostic apparatus, then being weighted and summed up. This process is also performed in the transmit beamformer in a similar manner, not only in the receive beamformer.

On the other hand, as a basic problem of the ultrasound imaging apparatus, it is known that lateral resolution is subject to constraints. Since transmitting and receiving of the ultrasound waves are performed by an array having a finite opening size, there is an impact of diffraction on the edge of the opening. If an infinitely long array is prepared, there is a possibility that the resolution is enhanced infinitely in the same manner as in the depth direction. In actual, however, a physical restriction in designing the apparatus, i.e., the length of the array, has hampered the enhancement of the lateral resolution. In recent years, it is attempted that the aforementioned fixed weight vector used for delaying, upon the delay-and-sum by the beamformer, is made to vary adaptively for the time-series transmit-receive data items, one by one, thereby obtaining an ultrasound image of higher definition, and this attempt is coming to attention. Accordingly, there is a possibility that this brings a marked improvement in the lateral resolution, being one of essential problems in the beamforming technique.

Particularly in recent years, an improved lateral resolution technique is being reported which is achieved by applying an adaptive signal processing technique including the MVDR method (Minimum Variance Distortionless Response; Capon method) that has been developed in the field of mobile communication, to the beamformer of the received data (Non Patent Documents 1 to 6). The technique described in those adaptive methods is implemented by adaptively varying complex components of the weight vector used for the delay-and-sum, on the basis of the correlative matrix of the received data. In other words, conventionally, the weight vector has been a fixed value, but in the adaptive method, the weight vector is obtained according to an operation with the use of the received signal, for each sample point in the time direction of the received signal, and the received signal is multiplied by thus obtained weight vector.

In the adaptive signal processing, similar to the conventional delay-and-sum method, distortion (aberration) of the ultrasound wavefront presents a significant challenge, the distortion being caused by inhomogeneous sound-velocity distribution in a medium and scattering from micro scatterers. Also in the adaptive signal processing, the apparatus sets the focus of the receive beamformer, assuming that the sound-velocity is constant and the medium is homogeneous. Therefore, when distortion exists in sound-wave propagation, there is a problem that an image appears blurred, or an image is formed on a position different from the actual position. In the conventional delay-and-sum method, correction of the wavefront distortion (wave aberration) is a long standing problem, and aberration correction technique utilizing cross-correlation processing has been studied. Also in the adaptive beamformer, it is a challenge to significantly change the image quality of an ultrasound image caused by inhomogeneity of the medium.

Following prior arts are known as describing techniques of a beamformer relating to the present invention; the Patent documents 1 to 3 regarding delay time adjustment using plural beams, the Patent document 4 regarding combined delay outputs, and the Patent document 5 regarding a technique for correcting wavefront distortion.

**Prior Art Document**

**Patent Document**

Patent Document 1
Japanese Unexamined Patent Application Publication No. 2010-82371
Patent Document 2
Japanese Unexamined Patent Application Publication No. 2010-63875
Patent Document 3
Japanese Unexamined Patent Application Publication No. 5-249088
Patent Document 4
Japanese Unexamined Patent Application Publication No. 2002-336249
Patent Document 5
Japanese Unexamined Patent Application Publication No. 7-303640

**Non Patent Document**

Non Patent Document 1

I. K. Holfort, et al., "Adaptive receive and transmit apodization for synthetic aperture ultrasound imaging", Proc. IEEE Ultrason. Symp., pp. 1 - 4 (2009) Non Patent Document 2
J. Capon: High-resolution frequency wavenumber spectrum analysis, Proc. IEEE, Vol. 57, pp. 1408 -1418, Aug. (1969)

Non Patent Document 3

F. Vignon et al.: Capon beamforming in medical ultrasound imaging with focused beams, IEEE Trans. Ultrason. Ferroelectr. Freq. Control, Vol. 55, No. 3, pp. 619 - 628 (2008)

Non Patent Document 4

J. F. Synnevag et al.: Benefits of Minimum-Variance Beamforming in Medical Ultrasound Imaging, IEEE Trans. Ultrason, Ferroelectr. Freq. Control, Vol. 56, No. 9, pp. 1868 - 1879 (2009)

Non Patent Document 5

I. K. Holfort et al.: Broadband minimum variance beamforming for ultrasound imaging, IEEE Trans. Ultrason. Ferroelectr. Freq. Control., Vol. 56, No. 2, pp. 314 - 325 (2009)

Non Patent Document 6

Z. Wang et al., "Time-delay- and time-reversal-based robust capon beamformers for ultrasound imaging IEEE Trans. Med. Imag., 24(10), pp. 1308 - 1322 (2005)

**Disclosure of the Invention**

**Problem to be solved by the Invention**

It is known that in an ultrasound diagnostic apparatus using an adaptive beamformer, beams are highly directive. However, actually, the medium of a test subject is inhomogeneous, and thus ultrasound signals being transmitted are reflected on a focus and scattered around the focus, resulting in that information regarding the object on the focus also exists around the focus. Beams with high directivity from the adaptive beamformer are incapable of acquiring ultrasound signals that exist around the focus, and this may cause a problem that an image quality is deteriorated. Furthermore, since the medium of the test subject is inhomogeneous, there may occur wavefront distortion due to the sound-velocity inhomogeneity, or the like. The wavefront distortion may cause reflected waves from the medium surrounding the focus to mix into the received sound wave. The reflected waves from the surrounding medium may become a noise signal correlated with the information from the focus, and there is a problem that this correlative noise may deteriorate the image quality.

Therefore, there is required a technique which allows the focus information scattered around the focus to be reproduced, in compatible with reducing unnecessary correlative noise from the medium surrounding the focus. In other words, an adaptive beamforming technique is demanded, being able to reduce the number of the ultrasound signals failing to be picked up, after scattered around the focus, highly robust against the correlative noise caused by the wavefront distortion, and provided with an image-quality compensation method with relatively small processing load.

An object of the present invention is to provide an ultrasound imaging apparatus that is capable of compensating for the image-quality deterioration caused by inhomogeneity of the test subject medium.

**Means to solve the Problem**

The ultrasound imaging apparatus of the present invention is provided with plural elements configured to receive ultrasound signals from a test subject, a delay unit configured to delay each of the received signals on the plural elements, in association with a position of a predetermined receive focus, and generate post-delay received signals, a peripheral information operator configured to acquire information items as to plural points within a region including the receive focus and a peripheral region thereof, respectively from the post-delay received signals, a peripheral information combiner configured to combine the information items acquired as to the plural points respectively, and generate a beamforming output by using the information being combined, and an image processor configured to use the beamforming output to generate image data.

**Effect of the Invention**

According to the present invention, it is possible to acquire ultrasound signals scattered around the focus, reduce of the number of the ultrasound signals failing to be picked up, achieve a highly robust property against the correlative noise caused by the wavefront distortion, and compensate for image-quality deterioration without significantly increasing the processing load.

**Brief Description of Drawings**

FIG. 1(a) is a perspective view illustrating a schematic configuration of the ultrasound imaging apparatus of the present embodiment, and FIG. 1(b) is a block diagram thereof;
FIG. 2 is a block diagram illustrating a configuration of the receive beamformer according to the first embodiment;
FIG. 3 is a block diagram illustrating a configuration of the receive beamformer according to the second embodiment;
FIG. 4 illustrates operations of the delay circuit according to the second embodiment;
FIG. 5(a) to FIG. 5(f) illustrate effects of the embodiments of the present invention;
FIG. 6 illustrates operations and effects of the signal processing in the second embodiment;
FIG. 7 is a block diagram illustrating a configuration of the peripheral information combiner in the receive beamformer according to the third embodiment;
FIG. 8 illustrates operations of the delay circuit according to the fourth embodiment;
FIG. 9 is a block diagram illustrating the receive beamformer according to the fourth embodiment;
FIG. 10(a) to FIG. 10(c) illustrate the steering vectors of the receive beamformer according to the fourth embodiment;
FIG. 11 is a block diagram illustrating a configuration of the receive beamformer according to the fifth embodiment;
FIG. 12 is a block diagram illustrating a configuration of a temporary storage in the receive beamformer according to the sixth embodiment;

FIG. 13 is a block diagram illustrating operations and effects of the signal processing in the sixth embodiment;

FIG. 14 is a block diagram illustrating a configuration of the temporary storage in the receive beamformer according to the seventh embodiment;

FIG. 15 is a block diagram illustrating operations and effects of the signal processing in the seventh embodiment;

FIG. 16 is a block diagram illustrating a configuration of the temporary storage in the receive beamformer according to the eighth embodiment;

FIG. 17 is a block diagram illustrating operations and effects of the signal processing in the eighth embodiment;

FIG. 18 is a block diagram illustrating a configuration of the temporary storage in the receive beamformer according to the ninth embodiment;

FIG. 19 is a block diagram illustrating operations and effects of the signal processing in the ninth embodiment;

FIG. 20 (a) to FIG. 20 (g) illustrate operations of the adjuster for setting the steering vectors according to the tenth embodiment;

FIG. 21 is a block diagram illustrating a configuration of the receive beamformer according to the eleventh embodiment;

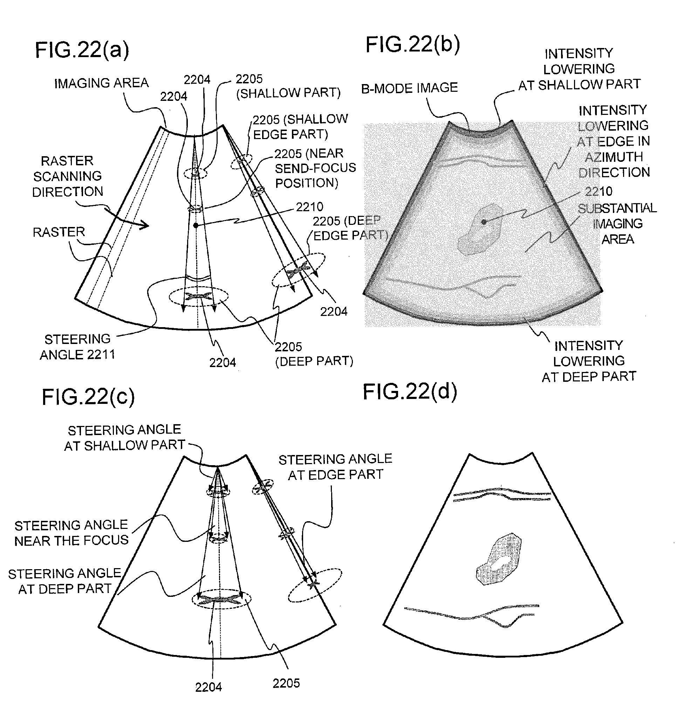

FIG. 22 (a) illustrates a mismatch between the signal spreading area by reflection at a focus (elliptic region 2205) and the point spread function 2204 based on the fixed steering angle, in association with the position of the ultrasound image, FIG. 22(b) illustrates the intensity distribution of the ultrasound image (B-mode image) obtained at the fixed steering angle, FIG. 22(c) illustrates relations between the point spread function 2204 at the steering angle set by the eleventh embodiment and the elliptic region 2205, and FIG. 22(d) illustrates the intensity distribution of the ultrasound image (B-mode image) obtained at the steering angle set in the eleventh embodiment;

FIG. 23(a) is a graph showing the signal strength or the intensity distribution of the B-mode image in the twelfth embodiment, and FIG. 23(b) is a graph showing the distribution of the steering angle obtained from the graph of FIG. 23(a) and a graph showing the distribution of a predetermined steering angle;

FIG. 24 is a perspective view of the console of the ultrasound imaging apparatus according to the present embodiments;

FIG. 25 is a perspective view of the console of another specific example and the monitor of the ultrasound imaging apparatus according to the present embodiments;

FIG. 26(a) illustrates the ultrasound images (B-mode images) showing six point scatterers when no wavefront distortion occurs in the ultrasound beam, and FIG. 26(b) illustrates the ultrasound images (B-mode images) of six point scatterers when wavefront distortion occurs in the ultrasound beam; and

FIG. 27 is a graph showing the intensity distribution of one point scatterer and surroundings thereof, with regard to the ultrasound images of FIG. 26.

## Modes for Carrying Out the Invention

Preferred embodiments of the present invention will be explained.

(First embodiment)

The ultrasound imaging apparatus of the first embodiment is provided with plural elements configured to receive ultrasound signals from a test subject, a delay unit configured to delay each of the received signals on the plural elements, in association with a position of a predetermined receive focus and generate post-delay received signals, a peripheral information operator, a peripheral information combiner, and an image processor. The peripheral information operator acquires information items as to plural points within a region including the receive focus and the peripheral region of the receive focus, respectively from the post-delay received signals. The peripheral information combiner combines the information items respectively acquired as to the plural points, and uses the information being combined to generate a beamforming output. The image processor uses the beamforming output to generate image data. Specific explanations will be provided in the following.

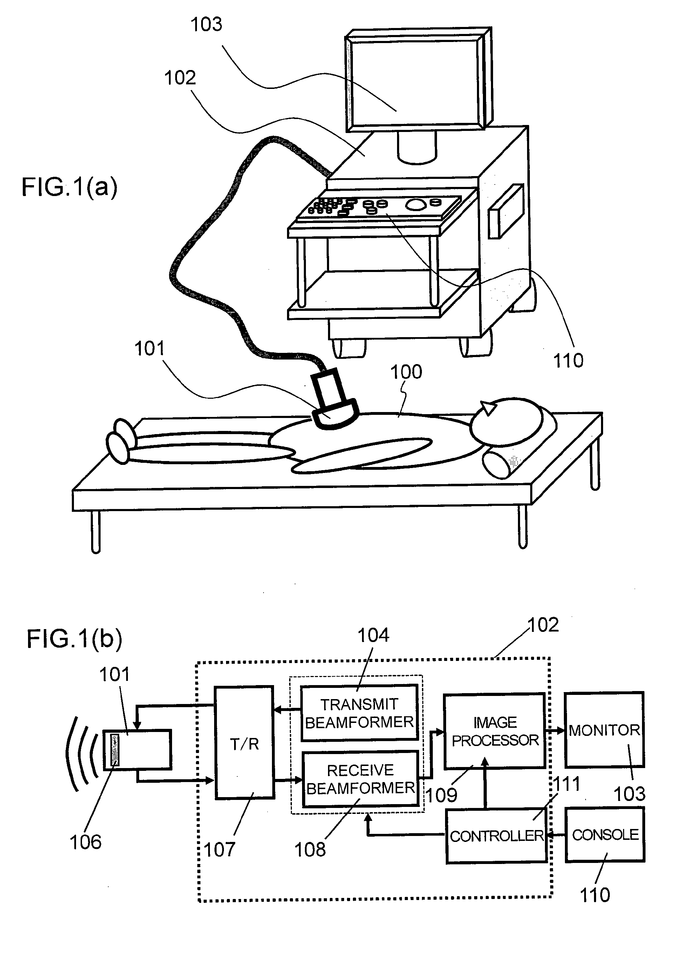

Firstly, with reference to FIG. 1(a) and FIG. 1(b), an overall configuration of the ultrasound imaging apparatus will be explained. FIG. 1(a) is a perspective view of the apparatus, and FIG. 1(b) is a block diagram schematically showing the inner configuration.

As illustrated in FIG. 1(a), the ultrasound imaging apparatus is provided with an ultrasound probe 101, an apparatus main body 102, a monitor 103, and a console 110. As illustrated in FIG. 1(b), the apparatus main body 102 incorporates a transmit beamformer 104, a transmit-receive separation circuit (T/R) 107, a receive beamformer 108, an image processor 109, and a controller 111 for controlling operations of those described above.

The ultrasound probe 101 is provided with plural elements (ultrasound transducers) 106 arranged in the form of an array. The transmit beamformer 104 generates signals for a transmission beam, and transfers the transmission beam to the ultrasound probe 101 via the transmit-receive separation circuit 107. The ultrasound probe 101 transmits ultrasound waves from the plural elements, directed to the inside of the test subject 100. The ultrasound probe 101 receives echo signals reflected inside the body. The received signals go through the transmit-receive separation circuit 107 again and subjected to beamforming operation, and the like, in the receive beamformer 108. The received signals after the beamforming operation is applied, are transferred to the image processor 109, and various image processes such as various filters, and a scan converter are executed on the received signals, and then, an ultrasound image is generated. The ultrasound image is transferred to the monitor 103, and displayed thereon.

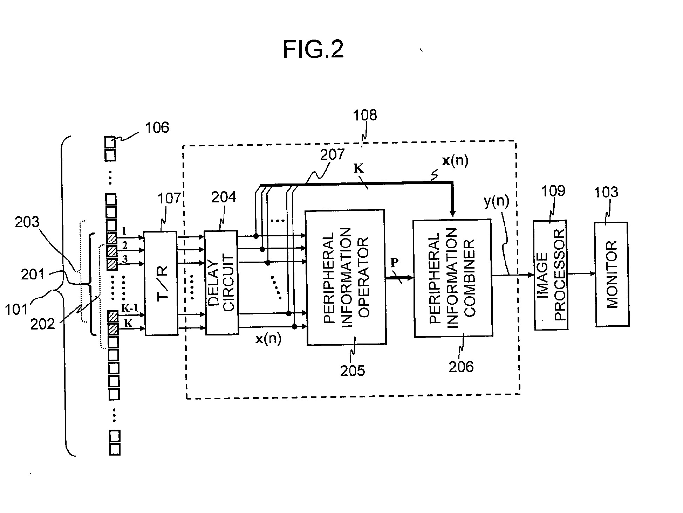

FIG. 2 is a block diagram illustrating the configuration of the receive beamformer 108. As shown in FIG. 2, the receive beamformer 108 incorporates a delay circuit 204, a peripheral information operator 205, a peripheral information combiner 206, and a bypass line 207 from the output of the delay circuit 204 to the peripheral information combiner. Independent circuits may configure those components in the receive beamformer 108, respectively. It is alternatively possible to configure such that a memory storing programs in advance and a CPU for reading and executing the programs implement the operations of those components.

In the receive beamformer 108 of the present embodiment, under the control of the controller 111, the active channel setter (not illustrated) sets an active channel in a part of the finite aperture of the ultrasound probe 101, so as to perform the receive beamforming process. In other words, the elements 106 within a partial range are assumed as the active channel 201, among the plural elements 106 constituting the ultrasound probe 101 which has received echoes associated with one transmit ultrasound beam, and the receive beamformer generates one line of image data (raster: beamforming output y(n)) in the ultrasound propagating direction, by using the received signals in the active channel 201. As illustrated in FIG. 2, the positions of the elements 106 in the channel are displaced gradually, thereby sequentially configuring the active channel 202, active channel 201, and active channel 203, and the rasters are generated respectively for the active channels 202, 201, and 203. Resultant arrangement of the rasters forms an ultrasound image.

The delay circuit 204 corresponds to a block provided as a previous stage of the peripheral information operator 205, configured to give a delay time to each of the received signals (received data) on the plural elements 106 constituting the active channel 201, in association with the element position, so as to perform processing to focus the received signals on a certain point virtually existing in a space. The delay time given to each of the elements 106 is prepared in advance, as a delay-time set, in association with the object positions in the space of the imaging target. The delay circuit 204 selects the delay-time set in response to the focal position being set, and gives different delay times to the respective received signals on the plural elements. With this configuration, it is possible to perform the focusing process covering the entire space of the imaging target.

The peripheral information operator 205 performs the operation to collect information items that are scattered around the receive focus due to the reflection from the receive focus. By way of example, the peripheral information operator 205 performs the adaptive signal processing, thereby obtaining adaptive weights as to two or more points, on the receive focus and in its periphery (peripheral region). With this configuration, it is possible to collect peripheral information with respect to the receive focus. It is to be noted that the periphery (peripheral region) corresponds to an area where signals are scattered due to the reflection of the signals on the receive focus, having a nearly elliptical shape (oval sphere in the three-dimensional case); the size being different depending on the conditions such as receive elements, a transmit frequency, a receive frequency, a ultrasound pulse waveform, the size and pitch of the element array, and a point spread function, roughly having a long axis spreading around $\pm 5°$ or less in the active channel direction, assuming as the central axis, the axis connecting the receive focus and the center point of the active channel, and a short axis in a length equivalent to or a little shorter than the active channel direction, in the ultrasound propagating direction orthogonal to the active channel direction. On this occasion, the focus is positioned at an intersection of the short axis and the long axis. At the same time, the periphery (peripheral region) corresponds to an area where the wavefront distortion causes that the reflected waves from the medium surrounding the focus exist in the received waves in mixed manner, and the reflected waves from the surrounding medium may become obvious in the received signals in the form of noise signals, being correlated with the information from the focus.

The peripheral information combiner 206 combines (compounds) the peripheral information items collected by the peripheral information operator 205 and obtains a final beamforming output y(n). By way of example, the peripheral information combiner 206 combines the adaptive weights of at least two points obtained by the peripheral information operator 205, and this combined weight being obtained is assigned on the signals of the elements 106, after the delay process is performed by the delay circuit 204, and weighted signals are subjected to beamforming, thereby combining the peripheral information items.

This finally obtained beamforming output y(n) is sequentially transferred to the image processor 109, raster by raster, respectively in association with the active channels 202, 201, and 203, and an ultrasound image is generated on the basis of thus combined beamforming output.

As described above, in the first embodiment, the peripheral information operator 205 collects the information items from the surrounding of the receive focus, and even though the adaptive beamformer with high directivity is used, it is possible to collect the information items reflected on the focus, and scattered in the surrounding thereof. Furthermore, by using thus collected peripheral information, an asymmetry property of the correlative signals is utilized, and this cancels (performs decorrelation of) the correlative noise caused by the wavefront distortion. Therefore, even when the medium of the test subject is inhomogeneous, it is possible to prevent the image quality from being deteriorated, resisting the influence from the correlative noise caused by the wavefront distortion, thereby achieving a highly robust ultrasound imaging apparatus.

(Second embodiment)

In the second embodiment, the peripheral information operator 205 in the ultrasound imaging apparatus of' the first embodiment performs the adaptive beamforming, thereby obtaining an adaptive weight as information.

The peripheral information operator 205 obtains the adaptive weights as to plural points, by using steering vectors that are directional vectors connecting a predetermined position on a surface of the array made up of the plural elements 106 as described in the first embodiment, and the plural points.

Furthermore, the peripheral information operator 205 incorporates a matrix operator configured to generate a covariance matrix by using the post-delay received signals, and a weight vector operator configured to obtain adaptive weight vectors as to the plural points, from the steering vectors. The peripheral information combiner 206 incorporates a weight combiner configured to sum the adaptive weights as to plural points obtained by the peripheral information operator 205 and generate a combined weight, and an inner-product operator configured to multiply the post-delay received signals by the combined weight to generate a beamforming output.

It is further possible to arrange a fixed apodization multiplier between the peripheral information operator 205 and the weight combiner, the fixed apodization multiplier being configured to multiply each of the adaptive weights as to the plural points obtained in the peripheral information operator, by a fixed weight being predetermined.

The inner-product operator multiplies each of the post-delay received signals by the combined weight, and thereafter sums the post-delay received signals, so as to generate the beamforming output.

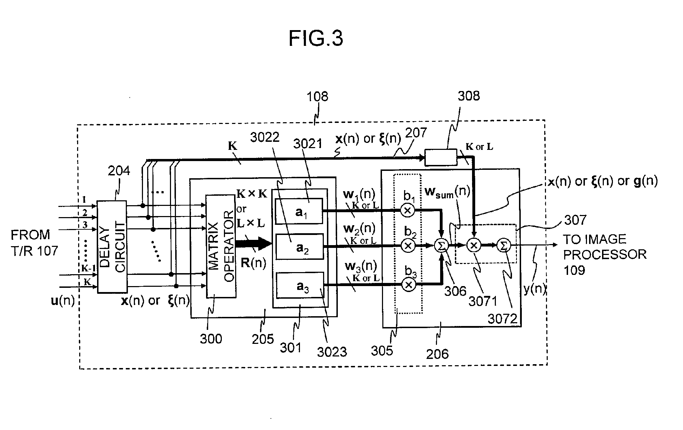

With reference to FIG. 3, the ultrasound imaging apparatus of the second embodiment of the present invention will be specifically explained. It is to be noted that a configuration similar to the first embodiment will not be tediously explained.

In the second embodiment, the peripheral information operator 205 obtains adaptive weights as to two or more points on the receive focus and in the periphery thereof, thereby collecting the peripheral information of the receive focus. Specifically, upon calculating the adaptive weight, plural steering vectors "a" are used so as to obtain the adaptive weight also as to a point at which the direction from the elements in the active channel of the ultrasound probe is displaced from the receive focus (i.e., a point at which the direction of the steering vector is different from the focus direction). The peripheral information combiner 206 combines the adaptive weights of two or more points obtained by the peripheral information operator 205, and uses thus obtained combined weight to perform beamforming process on the received signals. The steering vectors are directional vectors connecting the center position of the active channel and the points as described above. Details of the steering vectors will be explained with reference to the formula (4) described below. Hereinafter, more specific explanations will be given.

FIG. 3 is a block diagram illustrating a configuration of the receive beamformer 108 according to the second embodiment. Here, as a preferred embodiment of the present invention, there is illustrated an example to use an adaptive beamformer in which the adaptive signal processing technique is applied to the beamformer. It is to be noted here that the components other than the receive beamformer 108, in other words, the ultrasound probe 101, the transmit beamformer 104, and the transmit-receive circuit 107, being positioned in the previous stage of the receive beamformer 108, and the image processor 109 and the monitor 103 being positioned in the subsequent stage of the receive beamformer 108, are the same as those of the first embodiment. Therefore, tedious explanations and illustrations will not be given here.

As illustrated in FIG. 3, the peripheral information operator 205 in the receive beamformer 108 is provided with the matrix operator 300 and the adaptive weight operator 301. The adaptive weight operator 301 incorporates plural weight vector operators 3021, 3022, and 3023, and computes the weight vectors respectively as to the receive focus and the points surrounding thereof. In this example here, three weight vector operators 3021 to 3023 are arranged, and thus it is possible to obtain the weight vectors as to three points. In the present embodiment, however, the number of the weight vector operators is not limited to three, and any number may be applicable, as far as it is more than one.

The matrix operator 300 receives inputs from the delay circuit 204 (post-delay received data $x(n)$), and generates the spatial covariance matrix $R(n)$. After inputted in the adaptive weight operator 301, in each of the plural weight vector operators 3021, 3022, and 3023, the spatial covariance matrix calculates weight vectors $w_1(n)$, $w_2(n)$, and $w_3(n)$, as to the predetermined three points with different steering vector directions, out of the points including the receive focus and in the periphery thereof, and outputs the calculated weight vectors to the peripheral information combiner 206. Details of the steering vectors will be explained in the following.

The peripheral information combiner 206 is provided with the weight combiner 306 and the inner-product operator 307. The weight combiner 306 sums the plural weight vectors $w_1(n)$, $w_2(n)$, and $w_3(n)$ to combine the vectors, and generates a combined weight $w_{sum}(n)$. The inner-product operator 307 uses this combined weight $w_{sum}(n)$ to assign weight on the post-delay received data x(n) that is inputted via the bypass line 207, and then sums the data to obtain the beamforming output y(n). With this configuration, it is possible to obtain information in the form of one beamforming output, the information being acquired from the plural points within an identical raster and being different in the steering vector direction. Specifically the inner-product operator 307 incorporates a multiplier 3071 configured to multiply the post-delay received data x(n) by the combined weight $w_{sum}(n)$, and an adder 3072 configured to calculate the sum of the post-delay data after the multiplication.

If needed, the fixed apodization multiplier 305 may be added to the peripheral information combiner 206. The fixed apodization multiplier 305 multiplies the weight vectors $w_1(n)$, $w_2(n)$, and $w_3(n)$, by a predetermined fixed weight, so as assign the weight on the weight vectors.

The beamforming output y(n) obtained by the peripheral information combiner 206 is transferred to the image processor 109. The image processor 109 generates an ultrasound image on the basis of the beamforming result y(n).

In the following explanation, operations of each part will be explained in more detail, in the case where the received data is processed on each of the elements 106 in one active channel 201 in association with one-time transmission and receiving, so as to generate the beamforming data for one raster.

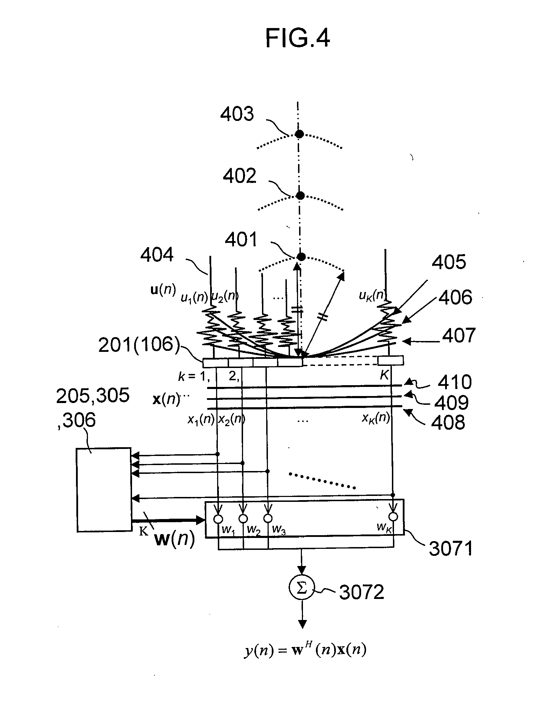

FIG. 4 illustrates the operations of the delay circuit 204, including some other parts. Firstly, K elements 106 constituting the active channel 201 receive K pieces of data. Hereinafter, K pieces of the received data is also referred to as K-channel received data u(n). The received data u(n) passes through the transmit-receive separation circuit 107, and enters the delay circuit 204. The delay circuit 204 gives to the received signals (received data 404) $u_1(n)$, $u_2(n)$, ..., $u_K(n)$ of K elements 106 constituting the active channel 201, delay time in such a manner as distributing the delay amount in the form of the concave shape 405 assuming one point 401 in the space as a center, in association with the positions of the elements 106, and then, obtains the post-delay received data $x_1(n)$, $x_2(n)$ , ..., $x_K(n)$. With this configuration, the focus 401 is virtually obtained. Here, "n" represents a certain time (snapshot time) in the time direction (the depth direction in the ultrasound propagating direction) of the ultrasound received signal. When the total number of samples in one raster is assumed as N, it is in the range of $0 < n < (N + 1)$. By shifting this snapshot time n, and also changing the concave shape 405 to a different concave shape (e.g., concave shapes 406 and 407), each of the focuses 402 and 403 is allowed to be obtained. With this configuration, the received signal 404 on each element 106 is delayed, focusing on a desired point, and time-series data items 408, 409, and 410 of the received signals (post-delay received data) with aligned wavefront are obtained.

If the number of the elements constituting the active channel 201 (the number of channels) is K, it is possible to express K post-delay received data items at a certain snapshot time n, as the vector x(n) on the left-hand side of the following formula (1).

[Formula 1]

$$\mathbf{x}(n) = \left[ x_1(n), x_2(n), ..., x_K(n) \right]^T \qquad \cdots \quad (1)$$

In the adaptive beamformer according to a conventional technique, this post-delay received data x(n) is inputted in the adaptive processor, so as to generate adaptive weight vector $w(n) = [w_1(n), w_2(n), ..., w_K(n)]^T$ being made up of weight values $w_K(n)$ for the respective K channels at the snapshot time n. Furthermore, this adaptive weight vectors w(n) and the post-delay received data x(n) are subjected to an inner-product operation in the inner-product operator, and the adaptive beamformer output y(n) at a certain snapshot time n is obtained. In the present invention, plural steering vectors "a" are used to obtain the adaptive weight vectors also for the periphery of the focal position.

As illustrated in FIG. 3, the post-delay received data vectors x(n) are inputted in the peripheral information operator 205. In the peripheral information operator, the matrix operator 300 firstly obtains the spatial covariance matrix R(n) according to the formula (2). Upon obtaining R(n), x(n) may be used as a real signal as it is, or it may be subjected to Hilbert transform or baseband modulation, so as to use the resultant complex data after the conversion. Here, a more general example will be explained, taking an example that x(n) is converted to complex data $\xi(n)$, and the spatial covariance matrix R(n) is obtained as expressed by the formula (2). R(n) in the formula (2) indicates an ensemble average of a product of the complex vector $\xi(n)$ expressed by the formula (3) and its (complex) conjugate transpose vector $\xi^H(n)$.

[Formula 2]

$$\mathbf{R}(n) = E[\xi(n)\xi^H(n)] = E\left\{ \begin{pmatrix} \xi_1(n)\xi_1^*(n) & \xi_1(n)\xi_2^*(n) & \cdots & \xi_1(n)\xi_k^*(n) \\ \xi_2(n)\xi_1^*(n) & \xi_2(n)\xi_2^*(n) & \cdots & \xi_2(n)\xi_k^*(n) \\ \vdots & \vdots & \ddots & \vdots \\ \xi_K(n)\xi_1^*(n) & \xi_K(n)\xi_2^*(n) & \cdots & \xi_K(n)\xi_K^*(n) \end{pmatrix} \right\}$$

$$= \frac{1}{N}\sum_{s=-S}^{S} \xi(n+s)\,\xi^H(n+s) \qquad \cdots \quad (2)$$

[Formula 3]

$$\xi(n) = [\xi_1(n), \xi_2(n), \ldots, \xi_K(n)]^T \qquad \cdots \quad (3)$$

In the formula (2), the ensemble average number N may indicate as a uniform mean as shown on the rightmost side of the formula (2), assuming the total number of samples before and after $\xi(n)$ of the target snapshot as N = 2S + 1 points. Another method for averaging in the time direction may be taken, the method multiplying each sample in the time direction by an arbitrary weight, such as a trapezoidal weight, and calculating the arithmetic mean. The spatial covariance matrix R(n) outputted from the matrix operator 300 is subsequently inputted into the adaptive weight operator 301.

The adaptive weight operator 301 that has received the spatial covariance matrix R (n) calculates weight vectors w(n) according to the MVDR method. In the present invention, plural steering vectors "a" are used to obtain the peripheral information of the focus. The steering vectors $a_p$ is expressed by the formula (4).

[Formula 4]

$$\mathbf{a}_p = \left[ \exp\{\psi_1(\theta_p, \phi_p, f_p)\}, \exp\{\psi_2(\theta_p, \phi_p, f_p)\}, \ldots, \exp\{\psi_K(\theta_p, \phi_p, f_p)\} \right]$$

$$= \left[ 1, \exp\left(-j\frac{2\pi}{\lambda_p} d \sin\theta_p\right), \exp\left(-j\frac{2\pi}{\lambda_p}\cdot 2d\sin\theta_p\right), \ldots \exp\left(-j\frac{2\pi}{\lambda_p}(K-1)d\sin\theta_p\right) \right]$$

$$\cdots \quad (4)$$

In the above formula (4), "p" represents the number of the steering vectors, and it is an integer satisfying $0 < p < P + 1$, when the total number is assumed as P. As shown in the formula (4), the steering vectors $a_p$ is a directional vector having K vector elements (from 0 to (K - 1)), the number being equal to the number of the active channels. The steering vector is expressed by a function of the receive frequency $f_p$ and the angle $(\theta_p, \varphi_p)$ formed by the normal vector direction on the surface of the elements 106 and the steering vector (hereinafter, referred to as "steering angle"). Here, "$\theta p$" represents an aperture angle from the normal vector, and "$\varphi_p$" represents a swivel angle from the array direction of the elements 106. When the array of the elements 106 in the ultrasound probe 101 is a one-dimensional (linear) array, the steering angle is expressed by the final form of the formula (4). Here, "$\lambda_p$" represents a wavelength of a sound wave associated with the frequency fp, and "d" represents a distance between the center of the elements 106 (pitch between the elements).

The weight vector w (n) obtained by the MVDR method, as to the direction of the steering vector $a_p$ as described above is calculated by the formula (5) in this example. Therefore, the weight vectors w(n) are computed as to the steering vectors $a_p$ being different respectively in the weight vector operators 3021 to 3023, thereby obtaining the adaptive weight vectors $w_1(n)$ to $w_P(n)$ corresponding to the number P of the steering vectors $a_p$ (i.e., the number of the weight vector operators 3021 to 3023).

[Formula 5]

$$\mathbf{w}_p(n) = \frac{\mathbf{R}^{-1}(n)}{\mathbf{a}_p^H \mathbf{R}^{-1}(n)\mathbf{a}_p} \qquad \cdots \ (5)$$

In the formula (5), R(n) represents the spatial covariance matrix at a certain snap shot n in the time direction, being generated by the formula (2), and the superscript "-1" represents an inverse matrix.

It is to be noted that the number P of the steering vectors may be any value, as for as it is an integer at least two. Any method for selecting the direction of the steering vector is applicable. In the present embodiment, it is assumed that the focus is positioned on the central axis of the channel 201; i.e., if the number of the active channels is an even number, it is positioned on the normal line (the normal line is orthogonal to the active channel surface) passing through the middle point between the K/2th and the (K + 2)/2th elements, out of the K elements 106 constituting the active channel 201. If the number of the active channels is an odd number, it is assumed that the focus is positioned on the normal line (the normal line is orthogonal to the active channel surface) passing through the center of the (K + 1)/2th element, out of the K elements constituting the active channel 201. It is desirable that one of the steering vectors faces the focus direction. Another (P-1) steering vectors are configured in such a manner as facing any directions within the region surrounding the focus in the imaging space. FIG. 3 illustrates the case where the total number of the steering vectors P = 3, but the total number of the steering vectors may be any number. The steering angle thereof may be for instance, at an equiangular pitch, or conform to a non-linear rule, or any irregular and random method may be taken for selecting the angle. By way of example, the configuration of FIG. 3 illustrates the case where P = 3, and the number of the weight operator outputs is three; that is, $w_1(n)$, $w_2(n)$, and $w_3(n)$.

As described above, since the delay process is performed in the delay circuit 204, the received data vector x(n), for instance, at a certain clock time n in the linear scanning becomes data with aligned wavefronts in the direction of $\theta = 0°$ being the direction of the normal vector. Therefore, in the formula (4), if it is assumed that $\theta_p = 0°$, the steering vectors $a_p$ are equal to $[1, 1... 1]^T$, and the adaptive weight vector w (n) in the focus direction is obtained. The adaptive weight vector w (n) in the focus direction corresponds to the adaptive weight vector w(n) obtained by the conventional MVDR method. In other words, if it is assumed that P = 1 and $\theta_p = 0°$ in the configuration of the present embodiment, it corresponds to the configuration of the adaptive beamformer using an ordinary MVDR method. The beamformer according to the present embodiment is different from the beamformer according to the ordinary MVDR method, in the point that various plural steering vectors $a_p$ are set, and adaptive weight vectors $w_1(n)$ to $w_P(n)$ are obtained for the respective vectors.

The present invention is not limited to the configuration that one of the plural steering vectors $a_p$ faces the focus direction (front direction) ($\theta_P = 0°$, $a_p = [1, 1... 1]^T$), but all the steering vectors may face the directions different from the focus direction. In particular, as a direction vector constituting the plural steering vectors $a_p$, the direction vector in the focus direction ($\theta_p = 0°$) is not used on purpose, and only the direction vector in the direction ($\theta_p < 0°$, $0° < \theta_p$) excluding the focus direction may be used. In this situation, the focus direction is not used on purpose, and this enhances the effect of the correlative noise canceling (decorrelation) more. Therefore, it is one of the preferred embodiments of the present invention.

The P adaptive weight vectors $w_1$n) to $w_P(n)$ outputted from the adaptive weight operator 301 are inputted into the peripheral information combiner 206. The peripheral information combiner 206 sums the P adaptive weight vectors $w_1(n)$ to $w_P(n)$ in the weight combiner 306, takes an arithmetic average, and calculate the combined weight vector $w_{sum}(n)$ as shown in the formula (6). By way of example, as shown in FIG. 3, the combined weight vector $w_{sum}(n)$ of the adaptive weight vectors when P = 3, are obtained as $w_{sum}(n) = \{w_1(n) + w_2(n) + w_3(n)\}/3$.

[Formula 6]

$$\mathbf{w}_{sum}(n) = \sum_{p=1}^{P} \mathbf{w}_p(n) = \frac{1}{P}\{\mathbf{w}_1(n) + \mathbf{w}_2(n) + \cdots \mathbf{w}_p(n) \cdots + \mathbf{w}_P(n)\} \qquad \cdots \ (6)$$

It is to be noted that in the stage prior to obtaining the combined weight vector $w_{sum}(n)$, a fixed apodization multiplier 305 may be provided, so as to multiply each of the weight vectors $w_p(n)$ by a fixed apodization. By way of example, it is possible to multiply the weight vectors by the fixed apodization bp having a distribution where a value of the adaptive weight vector $w_P(n)$ in the direction of $\theta = 0°$ is made larger and values in another directions are made smaller, and this is implemented by the computation as shown in the formula (7).
[Formula 7]

$$\mathbf{w}_{sum}(n) = \sum_{p=1}^{P} b_p \mathbf{w}_p(n) = \frac{1}{P} \left\{ b_1 \mathbf{w}_1(n) + b_2 \mathbf{w}_2(n) + \cdots b_p \mathbf{w}_p(n) \cdots + b_P \mathbf{w}_P(n) \right\} \quad \cdots \quad (7)$$

By way of example, in the configuration of FIG. 3, there are prepared fixed apodization weights $b_1$, $b_2$, and $b_3$ in the fixed apodization multiplier 305. In this situation, the combined weight outputted from the weight combiner becomes $w_{sum}(n) = \{b_1 w_1(n) + b_2 w_2(n) + b_3 w_3(n)\}/3$.

Next, the combined weight vector $w_{sum}(n)$ is inputted into the inner-product operator 307 within the peripheral information combiner 206. The inner-product operator 307 incorporates the multiplier 3071 and the adder 3072, performs the inner-product operation between the combined weight vector $w_{sum}(n)$ and the received data vectors x(n) transferred from the delay circuit 204 via the bypass line 207, as shown in the formula (8), and then obtains the beamforming output y(n). Specifically, the multiplier 3071 obtains the product of the weight vector and each channel element of the post-delay received data vectors (1 to K). The adder 3072 calculates a total sum of the K products obtained by the multiplier 3071, and obtains a final output of the peripheral information combiner 206 (beamforming output y(n), scalar value).
[Formula 8]

$$y(n) = \mathbf{w}_{sum}^{H}(n)\mathbf{x}(n) \quad \cdots \quad (8)$$

This beamforming output y(n) from the inner-product operator 307 is transferred to the image processor 109. The processing above is repeated for the N samples constituting one raster, from the first sample n = 1 to the final sample n = N, and then, N beamforming outputs y(n); y(1), y(2)... y(N), are transferred sequentially to the image processor 109. Consequently, the receive beamformer 108 completes the computations of the beamforming outputs y(n) for one raster in association with the active channel 201.

The beamforming outputs y(n) for one raster obtained by the formula (8), are acquired respectively for the active channels, shifting on the received array from the active channel 201 to the active channels 202, and 203, and transferred to the image processor 109, raster by raster. In the image processor 109, a scan converter associated with the scanning system of the ultrasound probe 101, arranges all the rasters and generates a two-dimensional image. In addition, backend image processing such as various filter processing, and computations by measurement applications are performed. Finally the monitor 103 displays an ultrasound image and a computational result of the measurement applications.

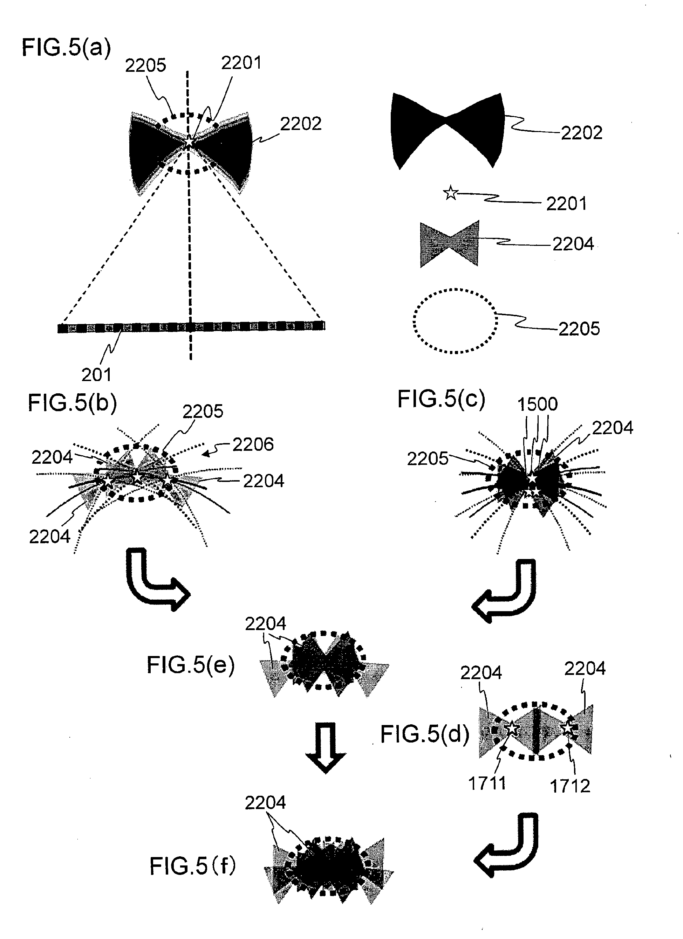

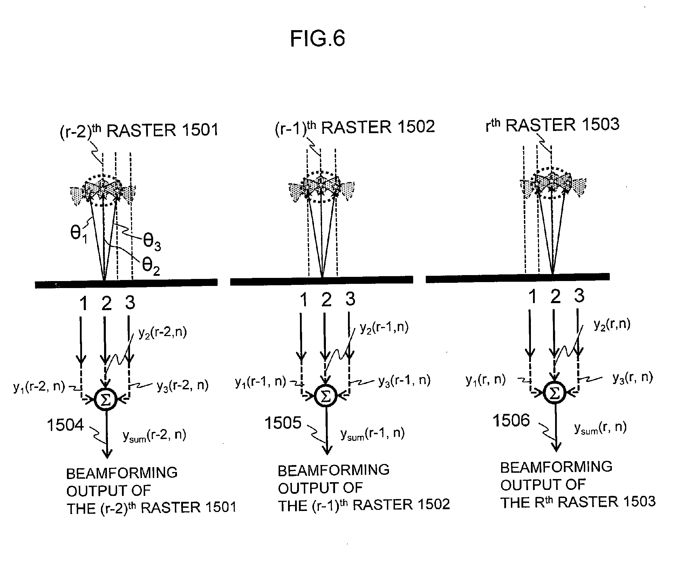

As described above, in the second embodiment, plural steering vectors $a_p$ are set, and the adaptive weight vectors $w_1(n)$ to $w_P(n)$ are obtained for the respective steering vectors, and combined weight vector $w_{sum}(n)$ obtained by combining those adaptive weight vectors is used to perform beamforming of the received data vectors x(n). Then, one beamforming output y(n) is obtained. With reference to FIG. 5 and FIG. 6, the effect will be explained.

FIG. 5(a) illustrates an example that data is received once from the active array 201, focusing on the receive focus 2201, and the receive beamforming is performed. When the receive beamforming is performed according to the conventional delay-and-sum method, signals in the region indicated by the point spread function 2202 around the receive focus 2201 may also be acquired by one-time receive beamforming. On the other hand, when adaptive beamforming is performed for this case, since the beam directivity is higher than the delay-and-sum method, the range of the signals being obtained by one-time receive beamforming is represented by the point spread function 2204, and it is smaller than the range represented by the point spread function 2202 of the delay-and-sum method. Here, it is conceivable that the signals of the object on the focus 2201 are reflected by the focus 2201 and distributed randomly in the periphery of the focus 2201 as indicated by the elliptic region 2205. This elliptic region 2205 corresponds to the periphery (peripheral region) of the first embodiment.

In the second embodiment, as described above, the adaptive weight vectors obtained by the adaptive beamformer, as to the respective plural points (here three points) having different steering angles are combined. This process is equivalent to allow the regions of the point spread function 2204 to overlap one another, the regions corresponding to the respective peripheries of the plural points as shown in FIG. 5(b). Therefore, in the second embodiment, the region 2206 obtained by the three displaced regions of the point spread function 2204 overlapping one another become the region allowing the signals therein to be acquired by one-time receiving. Therefore, it is possible to acquire most of the signals of the object, scattered in the elliptic region 2205 surrounding the focus 2201, and this allows reduction of the number of signals failing to be picked up.

By using the weight vectors having different steering angles, it is possible to utilize an asymmetry property of noise on the right side and noise on the left side of the central axis, being correlated with each other. In other words, adaptive weights having different steering angles are combined, thereby canceling (performing decorrelation of) the correlative noise signals in the received signals, and therefore, it is possible to selectively eliminate highly correlative noise signals, without failing to pick up the information of the signals on the focus.

According to the second embodiment, the effects as described above bring about improvements against the disadvantages such as; failing to pick up signals due to the sharp beam directivity of the adaptive beamformer, and being vulnerable to influence of the wavefront distortion. Therefore, this provides robustness, being impervious to such influence.

In the second embodiment, as shown in FIG. 6, the receive beamforming is performed every receiving (raster by rater). This may reduce the noise included in the beamforming outputs 1504, 1505, and 1506 respectively from the rasters 1501, 1502, and 1503, and attenuate the impact of the wavefront distortion. Therefore, an ultrasound image obtained by arranging those beamforming outputs of the respective rasters is a clear image with less noise, and with reduced impact of wavefront distortion caused by the influences such as sound-velocity inhomogeneous in the living body, a scatterer distribution, and body motion.

In the second embodiment, it is alternatively possible to perform spatial average operation using a subarray matrix, as one of other methods of the arithmetic algorithm in the aforementioned matrix operator 300. The subarray matrix $R^{\wedge}_{SUBl}$ in the spatial average operation is expressed by a product of the subspace vectors $\xi^{\wedge}_l(n)$ (Formula (10)) as shown in the formula (9). The subspace vector $\xi^{\wedge}_l(n)$ is a vector obtained by extracting a partial substance (L elements) from the post-delay received data (that is represented by generalized complex signal vector $\xi(n)$ here, but that may also be represented by a real vector $x(n)$, instead) in association with K active channels. Therefore, the total number of the subspace vectors is equal to (K - L + 1) (0 < 1 (1 is lower case of L) < (K - L + 1)).

[Formula 9]

$$\mathbf{R}^{\wedge}_{\mathrm{SUB}l}(n) = \xi^{\wedge}_l(n)\xi^{\wedge H}_l(n) \qquad \cdots (9)$$

[Formula 10]

$$\xi^{\wedge}_l(n) = [\xi_l(n), \xi_{l+1}(n), ..., \xi_{l+L-1}(n)]^T \qquad \cdots (10)$$

Samples are displaced one by one, establishing association between the main diagonal component of this subarray matrix and the main diagonal component of the spatial covariance matrix R(n), resulting in a spatial averaging process of the (K - L + 1) subarray matrixes, and the subarray spatial covariance matrix $R^{\wedge}(n)$ as shown in the formula (11) is obtained. When the adaptive weight operator 301 computes the subarray spatial covariance matrix $R^{\wedge}(n)$, it is used to substitute for R(n) in the above formula (5), and weight vectors $w_p(n)$ is computed as shown in the formula (12). It is to be noted that the output from the matrix operator 300 for this case is (L × L) in size, and the number of the elements constituting the weight vectors $w_p(n)$ becomes L.

[Formula 11]

$$\mathbf{R}^{\wedge}(n) = \frac{1}{N(K-L+1)} \sum_{s=-S}^{S} \sum_{l=1}^{K-L+1} \mathbf{R}^{\wedge}_{\mathrm{SUB}l} \qquad \cdots (11)$$

$$= \frac{1}{N(K-L+1)} \sum_{s=-S}^{S} \sum_{l=1}^{K-L+1} \xi^{\wedge}_l(n)\xi^{\wedge H}_l(n)$$

[Formula 12]

$$\mathbf{w}_p(n) = \frac{\mathbf{R}^{\wedge -1}(n)}{\mathbf{a}_p^H \mathbf{R}^{\wedge -1}(n)\mathbf{a}_p} \qquad \cdots \quad (12)$$

As another alternative example of the spatial averaging method, a forward and backward spatial averaging method may be employed. In this situation, the backward subarray matrix $R^\sim_{SUBl}(n)$ may be obtained by a product of the backward subspace vector $\xi^\sim_l(n)$ as shown in the formula (13). The backward subspace vector is expressed by the formula (14). According to the computation similar to the forward spatial averaging, the backward subarray spatial matrix $R^\sim(n)$ may be calculated as shown in the formula (15). Next, as shown in the formula (16), it is subjected to the arithmetic averaging together with the subarray spatial matrix $R^\wedge(n)$ for the case of the aforementioned forward spatial averaging, and finally the forward and backward subarray spatial covariance matrix $R_{FB}(n)$ may be obtained. Similar to the forward spatial averaging, this forward and backward subarray spatial covariance matrix $R_{FB}(n)$ is substituted for $R(n)$ in the formula (5) in the computations in the adaptive weight operator 301, thereby computing the weight vectors $w_p(n)$ as shown in the formula (17). Also in this case, the output from the matrix operator 300 is ($L \times L$) in size, and the number of elements constituting the weight vectors $w_p(n)$ is L.

[Formula 13]

$$\mathbf{R}^\sim_{SUBl}(n) = \xi^\sim_l(n)\xi_l^{\sim H}(n) \qquad \cdots \quad (13)$$

[Formula 14]

$$\xi^\sim_l(n) = \left[\xi_{K-l-1}(n), \xi_{K-l-2}(n), ..., \xi_{K-l-K}(n)\right]^T \quad \cdots \quad (14)$$

[Formula 15]

$$\mathbf{R}^\sim(n) = \frac{1}{N(K-L+1)} \sum_{s=-S}^{S} \sum_{l=1}^{K-L+1} \mathbf{R}^\sim_{SUBl}(n) \quad \cdots \quad (15)$$

$$= \frac{1}{N(K-L+1)} \sum_{s=-S}^{S} \sum_{l=1}^{K-L+1} \xi^\sim_l(n)\xi_l^{\sim H}(n)$$

[Formula 16]

$$\mathbf{R}_{FB}(n) = \frac{\mathbf{R}^\wedge(n) + \mathbf{R}^\sim(n)}{2} \qquad \cdots \quad (16)$$

[Formula 17]

$$\mathbf{w}_p(n) = \frac{\mathbf{R}_{FB}^{-1}(n)}{\mathbf{a}_p^H \mathbf{R}_{FB}^{-1}(n)\mathbf{a}_p} \qquad \cdots \quad (17)$$

Plural (P) weight vectors $w_p$ calculated in the formula (12) or in the formula (17) according to the spatial averaging method are transferred to the peripheral information combiner 206, similar to the case where the spatial averaging method is not employed. The weight combiner 306 sums the weight vectors, and calculates $w_{sum}$ (Formula (7), Formula (8)). Furthermore, the inner-product operator 307 performs the inner product calculation using the post-delay received data transferred via the bypass line 207, and eventually, the result is transferred to the image processor 109 as the beamforming data y(n).

In this example, the spatial averaging method is used under the condition that the number of the elements of the weight vectors $w_p$ is L. Therefore, in order to perform computations finally in the inner-product operator 307, a block is additionally required, so as to perform the computation that generates vectors g (n) having L components, out of $\xi$(n) having K components (Formula (18)). For example, as shown in FIG. 3, a dimensional compressor 308 may be arranged in the middle of the bypass line 207 and also in the previous stage of the inner-product operator 307, allowing the dimensional compressor 308 to generate the vectors g (n) having L elements, from $\xi$(n) having K elements. It is to be noted that the dimensional compressor 308 may be arranged within the peripheral information combiner 206.

The beamforming output y(n) using the spatial averaging method is expressed by the formula (19).

[Formula 18]

$$\mathbf{g}(n) = \sum_{l=1}^{K-L+1} \hat{\xi}_l(n) \qquad \cdots (1\ 8)$$

[Formula 19]

$$y(n) = \mathbf{w}_{\text{sum}}^H(n)\mathbf{g}(n) \qquad \cdots (1\ 9)$$

As described above, the matrix operator 300 performs the spatial averaging process using the subarray matrix, and produces an effect that restrains correlated noises being included in the received ultrasound signals. Therefore, the spatial averaging process using the subarray matrix is combined with the configuration for synthesizing plural adaptive weight vectors around the focus, thereby obtaining an ultrasound image that is much less influenced by noise.

In the second embodiment as described above, the MVDR is taken as an example for explaining the adaptive beamforming method. However, the algorithm applied in the adaptive weight operator 301 may be any other algorithm as far as it uses the spatial covariance matrix calculated in the matrix operator 300. Any other methods may be applicable, such as the MMSE method, APES method, Eigenspace-MV method (ESMV, EIBMV) using the spatial covariance matrix and its eigenvalues and eigenvectors, ESPRIT method, and MUSIC method.

(Third embodiment)

The third embodiment is different from the second embodiment in the point that the peripheral information combiner 206 multiplies the post-delay received signals, by the adaptive weights respectively for the plural points obtained by the peripheral information operator 205, and plural inner-product operators for generating pre-synthesis beamforming outputs respectively for the plural adaptive weights, and an output combiner are provided. The output combiner sums and combines the pre-synthesis beamforming outputs respectively as to the plural adaptive weights, and generates a beamforming output to be used for generating image data.

It is further possible to arrange between the peripheral information operator 205 and plural inner-product operators, a fixed apodization multiplier configured to multiply the adaptive weights for the respective plural points obtained by the peripheral information operator 205, by fixed weights being predetermined respectively. It is alternatively possible to arrange between the plural inner-product operators and the output combiner, the fixed apodization multiplier configured to multiply the plural post-delay received signals for the respective adaptive weights, by the fixed weights being predetermined respectively. It is further alternatively possible to arrange between the plural multipliers and the plural adders constituting the plural inner-product operators, the fixed apodization multiplier configured to multiply the plural signals after multiplication, by fixed weights being predetermined respectively.

The peripheral information combiner 206 combines the information items that is obtained by the peripheral information operator 205 from the received signals in one active channel, and generates a final beamforming output that is used by the image processor to generate an image. By way of example, the peripheral information combiner 206 sums the pre-synthesis beamforming outputs for the respective plural adaptive weights, obtained by the peripheral information operator 205 from the received signals in one active channel, and generates a final beamforming output that is used by the image processor 109 with respect to each active channel, so as to generate an image.

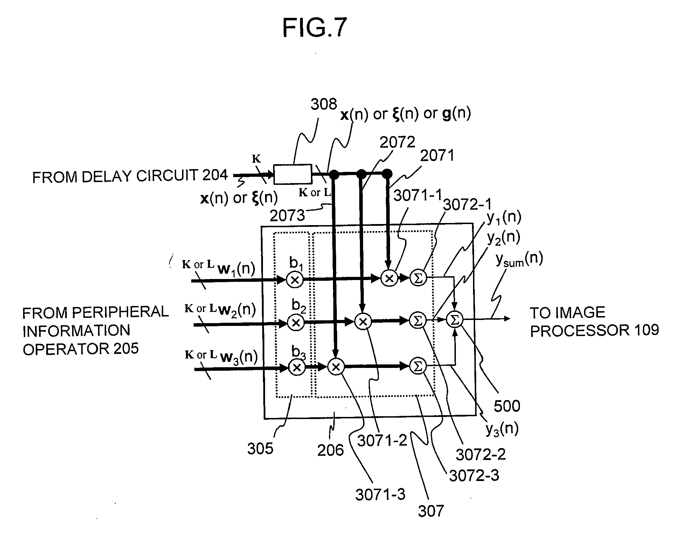

With reference to FIG. 7, the ultrasound imaging apparatus according to the third embodiment of the present invention will be explained specifically. In the third embodiment, since only the configuration of the peripheral information combiner 206 is different from the second embodiment, only the different part will be explained without tediously explaining the remaining parts.

In the second embodiment as shown in FIG. 3, the peripheral information combiner 206 combines the plural adaptive weight vectors $w_1(n)$, $w_2(n)$, and $w_3(n)$ by the weight combiner 306, and with the use of thus obtained combined weight $w_{sum}(n)$, the beamforming summation process is performed on the post-delay received data x(n). Since this is a linear operation, processing of the weight combining and processing of the beamforming summation may be transposed. The plural inner-product operator 307 of the third embodiment, as shown in FIG. 7, incorporates the plural multipliers (3071-1, 3071-2, and 3071-3), and the plural adders 3072-1, 3072-2, and 3072-3 configured to calculate the sum of the elements after the multiplication (post-delay data) to obtain the pre-synthesis beamforming output, respectively for the plural adaptive weight vectors $w_1(n)$, $w_2(n)$, and $w_3(n)$. A set of one multiplier 3071 and one adder 3072 configures one inner-product operator for each of the adaptive weight vectors. Preparing this set more than one configures plural inner-product operators 307. The post-delay received data x(n) is inputted in the multipliers 3071-1, 3071-2, and 3071-3, respectively via the bypass inputs 2071, 2072, and 2073. The output combiner 500 is arranged in the subsequent stage of the plural inner-product operators 307.

With this configuration, in the plural inner-product operators 307, the bypass input 2071 is subjected to the beamforming summation by using the weight vectors $w_1(n)$, the bypass input 2072 is subjected to the beamforming summation by using the weight vectors $w_2(n)$, and the bypass input 2073 is subjected to the beamforming summation by using the weight vectors $w_3(n)$. Then, plural pre-synthesis beamforming outputs $y_1(n)$, $y_2(n)$, and $y_3(n)$ respectively in association with the steering vectors are calculated (Formula (20) in the following). In the output combiner 500 in the subsequent stage, arithmetic averaging of the beamforming outputs $y_1(n)$, $y_2(n)$, and $y_3(n)$ respectively associated with the steering vectors is calculated, and the combined beamforming output $y_{sum}(n)$ is obtained as shown in the formula (21).
[Formula 20]

$$y_p(n) = \mathbf{w}_p^H(n)\mathbf{x}(n) \qquad \cdots (20)$$

[Formula 21]

$$y_{sum}(n) = \sum_{p=1}^{P} y_p(n) = \frac{1}{P}\left\{ y_1(n) + y_2(n) + \cdots y_p(n) \cdots + y_P(n) \right\} \qquad \cdots (21)$$

As illustrated in FIG. 7, also in the present embodiment, the fixed apodization multiplier 305 may be added. In FIG. 7, the fixed apodization multiplier 305 is placed in the previous stage of the inner-product operator 307. However, the position of the fixed apodization multiplier 305 is not limited to the place as shown in FIG. 7. Since the operation is a linear operation, it is obviously possible to add the fixed apodization multiplier 305 between the inner-product operator 307 and the output combiner 500, or to add the fixed apodization multiplier 305 between the multipliers 3071-1 to 3071-3 and the adders 3072-1 to 3072-3 in the inner-product operator. In any of the above cases, the final combined beamforming output $y_{sum}(n)$ is expressed by the formula (22).
[Formula 22]

$$y_{\mathrm{sum}}(n) = \sum_{p=1}^{P} b_p y_p(n) = \frac{1}{P}\left\{b_1 y_1(n) + b_2 y_2(n) + \cdots b_p y_p(n) \cdots + b_P y_P(n)\right\}$$

$$\cdots \quad (2\,2)$$

As described above, the second embodiment is different from the third embodiment in the point that the weights are combined and thereafter the inner-product operation is performed; or the beamforming outputs after the inner-product operation are combined. On the other hand, the final output is identical since this is a linear operation, and y(n) in the formula (19) being the final beamforming output of the second embodiment becomes a value being equal to the final combined beamforming output $y_{\mathrm{sum}}(n)$ of the third embodiment.

(Fourth embodiment)

In the fourth embodiment, the delay unit (delay circuit 204) generates post-delay received signals respectively for different plural receive focuses. The peripheral information operator 205 and the peripheral information combiner 206 acquire information items as to the receive focus and plural points in the periphery of the receive focus, as to each of the plural receive focuses, and generates a beamforming output. By way of example, the delay unit (delay circuit 204) is prepared more than one, and generates the post-delay received signal as to each of the receive focuses being different for the plural delay units, respectively. The peripheral information operator 205 and the peripheral information combiner 206 are arranged for each of the delay units, acquire information from the post-delay received signal generated by the delay unit for each receive focus, and generates the beamforming output.

On this occasion, the positions of the plural receive focuses of the received signals in the active channel at a certain point of time, may partially overlap the positions of the plural receive focuses for the active channel at a different point of time.

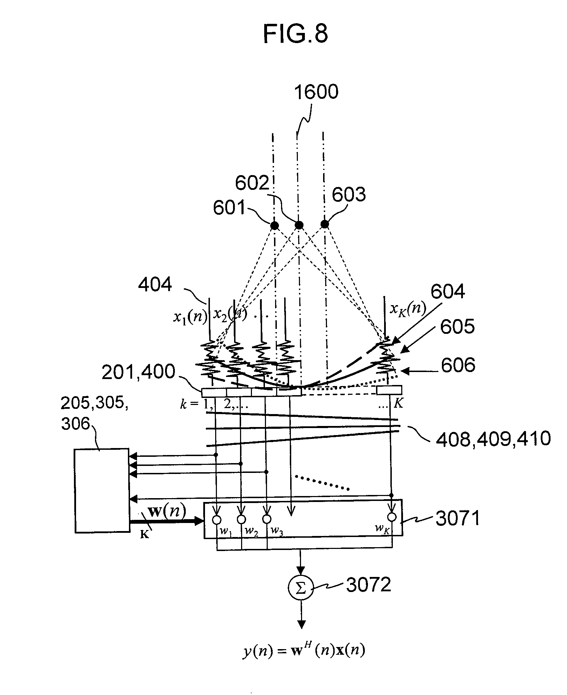

With reference to FIG. 8 to FIG. 10, the ultrasound imaging apparatus according to the fourth embodiment of the present invention will be specifically explained. The configurations similar to the second and the third embodiments may not be tediously explained, and only the different portions will be explained.

In the second and the third embodiments, explanations are provided for the case that the focus is positioned on the central axis (the normal line passing through the active channel center, with respect to the active channel surface) for every received signal. In the fourth embodiment, as illustrated in FIG. 8, plural points are set as the focuses at a certain depth for every received signal. By way of example, the focuses are set respectively on the points 601 and 603 on the axes in proximity to and displaced from the central axis 1600, on both sides of the point 602 on the central axis, and information of the test subject 100 is collected. Configuring a distribution of the delay times of the received signals of the K elements 106 constituting the active channel, in the form of concave 604, 605, and 606 respectively centering on the focus 601, 602, and 603, allows the signals received for one time, to focus on the plural focuses 601, 602, and 603.

In the present embodiment, the post-delay data obtained as to each of the plural focuses 601, 602, 603 at a certain depth (at a certain snapshot time n) is subjected to arithmetic operation for collecting and combining the periphery information.

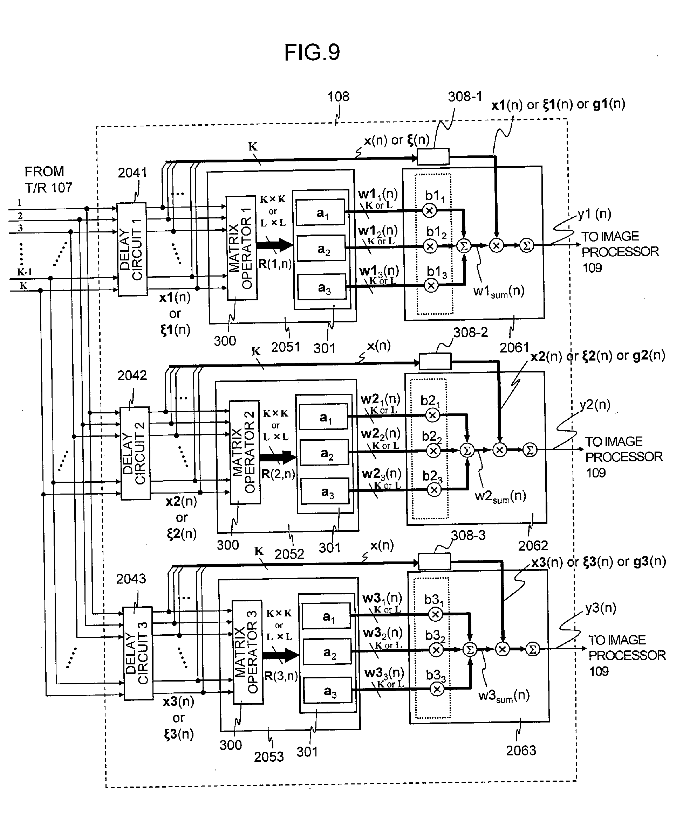

FIG. 9 is a block diagram illustrating the receive beamformer 108 according to the fourth embodiment. As shown in FIG. 8, in the present embodiment, in order to set plural focuses 601, 602, and 603 simultaneously at a certain snapshot time, the receive beamformer 108 is provided with the delay circuits 2041, 2042, and 2043, the peripheral information operators 2051, 2052, and 2053, and the peripheral information combiners 2061, 2062, and 2063, the number of which is the same as the number of the focuses. The configurations of the peripheral information operators 2051, 2052, and 2053, and the peripheral information combiners 2061, 2062, and 2063 are the same as those of the peripheral information operator 205 and the peripheral information combiner 206 in the second embodiment. Therefore, the number of the beamforming outputs y(n) is equal to the number of the focuses, and in the example of FIG. 9, there are three beamforming outputs, $y_1(n)$ $y_2(n)$, and $y_3(n)$.

The signal processing operations of the fourth embodiment will be explained. The delay circuit 2042 forms a delay concave surface to obtain the focus 602 on the central axis 1600 of the active channel at a certain snapshot time n, the delay circuits 2041 and 2043 form the delay concave surfaces respectively for obtaining the focuses 601 and 603 on the axes displaced from the central axis 1600, and then the delay circuits generate the post-delay received data.

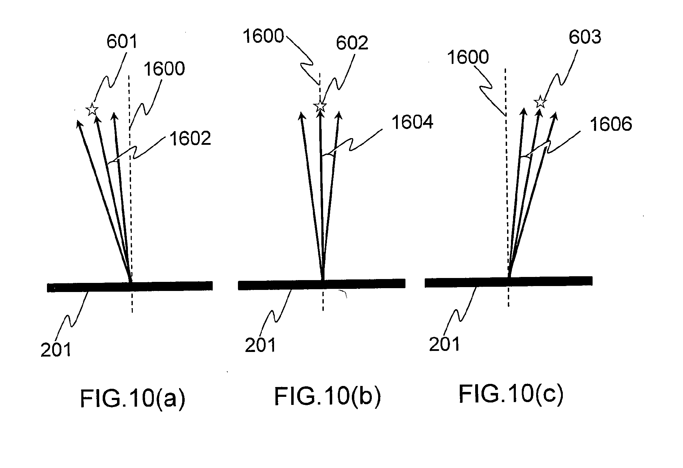

As shown in FIG. 10(a), the peripheral information combiner 2051 performs the operations as explained in the second embodiment, on the post-delay received data from the delay circuit 2041, so as to calculate the weight vectors $w1_1(n)$, $w1_2(n)$, and $w1_3(n)$ respectively for the focus 601 and two points forming the steering angle 1602 with respect to the direction of the focus 601. The peripheral information combiner 2061 combines the weight vectors $w1_1(n)$, $w1_2(n)$,

and w1$_3$(n), and then uses thus combined weight vector w1$_{sum}$(n) to obtain the beamforming output y1(n).

As shown in FIG. 10(b), the peripheral information combiner 2052 performs operations on the post-delay received data from the delay circuit 2042, and calculates the weight vectors w2$_1$(n), w2$_2$(n), and w2$_3$(n) respectively for the focus 602 and the two points forming the steering angle 1604 with respect to the direction of the focus 602. The peripheral information combiner 2062 combines the weight vectors w2$_1$(n), w2$_2$(n), and w2$_3$(n), so as to use thus combined weight vector w2$_{sum}$(n) to obtain the beamforming output y2 (n) .

Similarly, as shown in FIG. 10 (c), the peripheral information combiner 2053 performs operations on the post-delay received data from the delay circuit 2043, and calculates the weight vectors w3$_1$(n), w3$_2$(n), and w3$_3$(n) respectively for the focus 603 and the two points forming the steering angle 1606 with respect to the direction of the focus 603. The peripheral information combiner 2063 combines the weight vectors w3$_1$(n), w3$_2$(n), and w3$_3$(n), and uses thus combined weight vector w3$_{sum}$(n) to obtain the beamforming output y3(n).

Those beamforming outputs y1$_{sum}$(n), y2$_{sum}$(n), and y3$_{sum}$(n) are transferred to the image processor 109.

The processing above allows acquisition of ultrasound image data of the three focuses 601, 602, and 603, by using just one-time transmit-receive signals to and from the active channel 201, for instance. This processing indicates that when the direction of the focus 602 is assumed as the main beam direction, the image may be generated also in the sub-beam directions (the directions of the focuses 601 and 603). In other words, when the configuration of the fourth embodiment of the present invention is employed, i.e., to acquire signals of the object scattered around the focus, it is found that the configuration of the present invention is applicable to the technique such as the sub-beam processing and parallel beamforming. Therefore, there are obtained effects such as reduction of failing to pick up the signals and reduction of noise.

Furthermore, this process is also applicable to the plural active channels 201, 202, and 203. By way of example, the focus is set for the active channels 202 and 203 on the same point as the focus 602 for the active channel 201, in such a manner as viewing in the slant direction from the active channels 202 and 203, thereby newly acquiring the data regarding the focus 602. Therefore, as for the plural active channels 201, 202, and 203, data items acquired by viewing the identical point 602 from different directions are obtained respectively, and then, the obtained data items are combined and overlapped one another. This process is referred to as synthetic aperture (combined aperture) process. That is, by using the configuration of the fourth embodiment, the technique of the present invention for acquiring signals of the object, scattered around the focus, is applicable to the synthetic aperture process.

FIG. 8 to FIG. 10 illustrate the example where the number of the focuses at the same depth is three (N$_{focus}$ = 3), but the number of the focuses may be any number at least two. By way of example, it is possible to set sixteen focuses at the same depth. On this occasion, there are arranged sets of the delay circuit, peripheral information operator, and peripheral information combiner, the number of the sets being equal to the number of the focuses.

According to the present embodiment, an effect of the present invention, that is, reduction of the wavefront distortion, may be produced also in the off-axis beamforming technique focusing on an off-axis point, the parallel beamforming, and the synthetic aperture beamforming.

The internal configurations of the peripheral information operators 2051 to 2053 and the peripheral information combiners 2061 to 2063 of the present embodiment as shown in FIG. 9 are the same as those illustrated in FIG. 3. However, any configuration may be applicable as far as it is within the scope of the present invention. By way of example, the peripheral information combiner 206 of the third embodiment as shown in FIG. 7 may be employed. It is alternatively possible to employ the configurations of the peripheral information operator 205 and the peripheral information combiner 206 of the fifth embodiment to the eighth embodiment, which will be described in the following.

(Fifth embodiment)

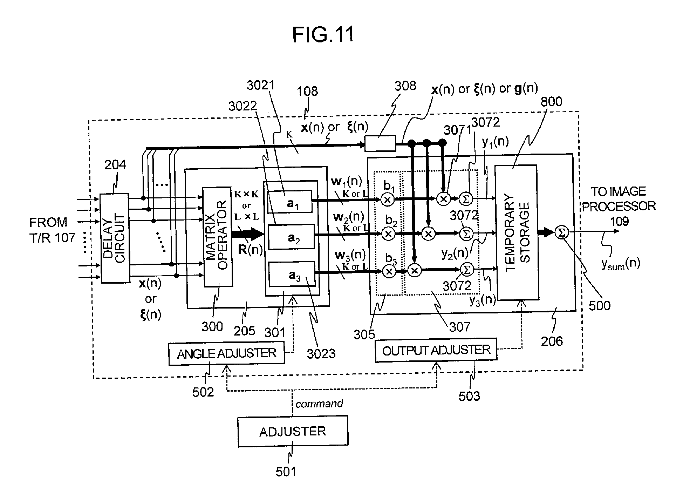

In the fifth embodiment, a storing unit (temporary storage 800) is arranged between the plural inner-product operators of the third embodiment and the output combiner, the storage being configured to store the pre-synthesis beamforming outputs respectively for the plural adaptive weights generated by the respective inner-product operators. With reference to FIG. 11, the ultrasound imaging apparatus according to the fifth embodiment of the present invention will be explained specifically. The configuration of the fifth embodiment is the same as that of the third embodiment, but they are different in the point that the temporary storage 800 is provided between the plural inner-product operators 307 and the output combiner 500 in the peripheral information combiner 206. In the third embodiment, as shown in FIG. 7, the signals to be subjected to the beamforming are flowing sequentially with time, from the time being received at the active channel until reaching the image processor 109. Therefore, the synthesizing process in the output combiner 500 is limited to the process for synthesizing the beamforming outputs y$_1$(n), y$_2$(n), and y$_3$(n), respectively obtained by the plural inner-product operators 307 as to the plural steering vectors of each data, in the raster currently under the beamforming process and at the current snapshot time n.

In the fifth embodiment, there is provided the temporary storage 800 as a memory configured to store the beamforming outputs y$_1$(n), y$_2$(n), and y$_3$(n) immediately before the output combiner 500. The temporary storage 800 stores the beamforming outputs $y_1(n)$, $y_2(n)$, and $y_3(n)$ obtained by the plural inner-product operators 307, every updating at the snapshot n, or every updating of the raster. With this configuration, the output combiner 500 is allowed to read and combine the beamforming outputs $y_1(n)$, $y_2(n)$, and $y_3(n)$ stored in the temporary storage 800, and it is possible to combine the beamforming outputs at various time samples within an identical raster, or combine the beamforming outputs across different rasters.

Furthermore, those beamforming outputs $y_1(n)$, $y_2(n)$, and $y_3(n)$ are results of the beamforming using the weight vectors $w_1(n)$, $w_2(n)$, and $w_3(n)$ obtained as to different steering vectors at every snapshot time n. Therefore, the beamforming outputs $y_1(n)$, $y_2(n)$, and $y_3(n)$ indicate beamforming outputs in the respective steering vector directions. This allows the output combiner 500 to select any combination of the beamforming outputs among different rasters and among different steering vector directions, and combine thus selected beamforming outputs. The beamforming outputs to be selected among the different steering vector directions may be predetermined, or set by the operator.

As illustrated in FIG. 11, the receive beamformer 108 may also be provided with an angle adjuster 502 and a memory output adjuster 503. The angle adjuster 502 takes control of setting the steering vector directions, the number of steering vectors, and the density thereof, in the weight vector operators 3021, 3022, and 3023 in the adaptive weight operator 301. The memory output adjuster 503 designates the timing (clock) for reading and transferring the beamforming output from the temporary storage 800 to the output combiner 500, and assigns in the memory, the address of the beamforming output to be read out. It is further possible to provide an adjuster 501 on an upper level, configured to adjust the parameters of both the angle adjuster 502 and the memory output adjuster 503 in conjunction with each other.

The peripheral information combiner 206 provided with the temporary storage 800 of the present embodiment may substitute for the peripheral information combiners 2061, 2062, 2063 of the fourth embodiment. This configuration allows synthesis of the beamforming outputs of different time samples as to each of the plural focuses in an identical raster, or synthesis of the beamforming outputs across different rasters, and the like.

(Sixth embodiment)

In the sixth embodiment, the output combiner 500 acquires and sums the pre-synthesis beamforming outputs different in the time direction, from the storing unit (temporary storage 800) and the plural inner-product operators, so as to generate a beamforming output.

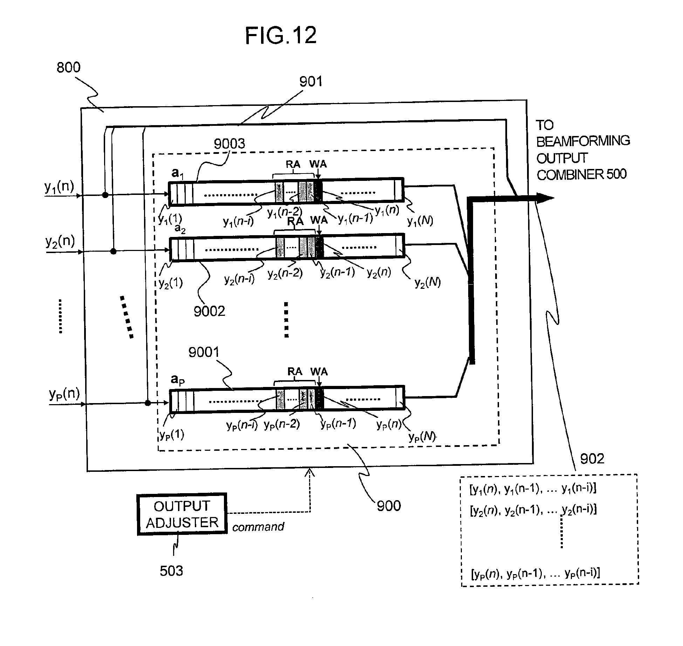

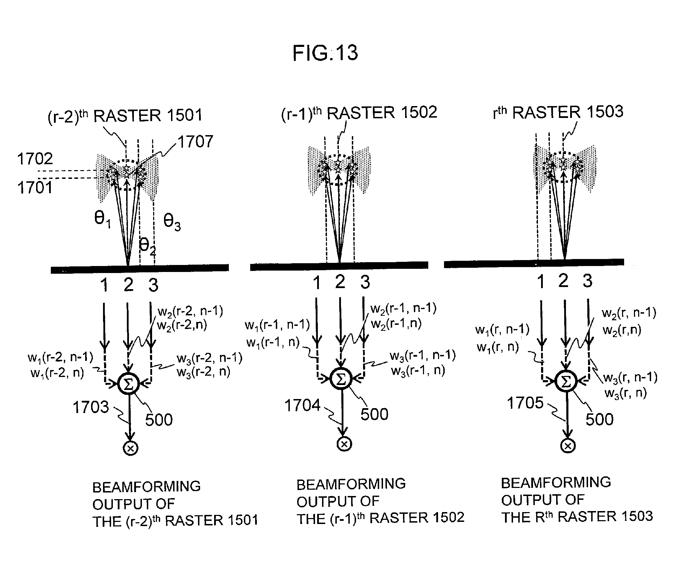

With reference to FIG. 12 and FIG. 13, the ultrasound imaging apparatus according to the sixth embodiment of the present invention will be explained specifically. Similar to the fifth embodiment, the ultrasound imaging apparatus of the present embodiment is provided with the temporary storage 800, but the internal configuration of the temporary storage 800 is different from the fifth embodiment.

In the present embodiment, the temporary storage 800 is provided with a memory 900, and a bypass line 901 for the beamforming outputs (hereinafter, also referred to as "post-beamforming data") $y_1(n)$ to $y_P(n)$. The memory 900 has a storage area (address) for storing (P × N) post-beamforming data items, that is, post-beamforming items $y_1(n)$ to $y_P(n)$ for every N snapshots in the time direction, in association with each of the P steering vectors. Specifically, as shown in FIG. 12, P memory areas 9001, 9002, and 9003 are prepared in the memory 900, each including N addresses schematically illustrated. Since other configurations are the same as those of the fifth embodiment, tedious explanations will not be provided.

Operations of the temporary storage 800 of the present embodiment are described as the following. The temporary storage 800 receives the post-beamforming data items $y_1(n)$ to $y_P(n)$ from the plural inner-product operators 307, at a certain snapshot time n. Those post-beamforming data items $y_1(n)$ to $y_P(n)$ are transferred to both the memory 900 and the bypass line 901. In the memory 900, the post-beamforming data items $y_1(n)$ to $y_P(n)$ are stored in a predetermined write address (WA) in the memory areas 9001, 9002, and 9003, respectively. Simultaneously, the post-beamforming data items $y_1(n-1)$ to $y_P(n-1)$ ... $y_1(n-i)$ to $y_P(n-i)$ at the sample times n-1 to n-i, stored in the read address (RA) are outputted. Those readout outputs at the sample times n-1 to n-i and the post-beamforming data items $y_1(n)$ to $y_P(n)$ passing through the bypass line at the current snapshot n are combined as an output, and this output is transferred from the output bus 902 to the output combiner 500.

In the configuration of the present embodiment, even at the sample time n, the beamforming outputs of the plural steering vectors 1 to P are outputted, combining the information items at the time (n-1) ... (n-i), i.e., being obtained by going back to $y_1(n-1)$ to $y_P(n-1)$ ... $y_1(n-i)$ to $y_P(n-i)$, with the current post-beamforming data items $y_1(n)$ to $y_P(n)$, and this allows the output combiner in the subsequent stage to combine all of those outputs together.

The information stored in the memory 900 is configured to be overwritten every time the raster is updated, so as to overwrite the information sequentially by the post-beamforming data in a new raster.

As shown in FIG. 13, this configuration allows the post-beamforming data items $y_1(n)$ to $y_P(n)$ ... $y_1(n-i)$ to $y_P(n-i)$ at the plural sample times from n to (n-i) to be combined, and it is possible to obtain the beamforming outputs 1703, 1704, and 1705, being acquired by synthesizing the beamforming results of plural time samples, respectively in the same rasters 1501, 1502, and 1503. This allows collecting the data items 1701 and 1702 inhomogeneously scattered in the time direction, and compensate for the image quality deterioration of the ultrasound image in the time direction (ultrasound wave propagating direction).

It is to be noted that the read address (RA) may be determined in advance, or an output adjuster 503 may be provided and configured to designate the read address. In the latter case, the output adjuster 503 may transfer to the temporary storage 800, for instance, the information regarding which data is outputted, out of the current post-beamforming data items $y_1(n)$ to $y_P(n)$ and the previous data held in the memory, and a signal for controlling the output timing and the output data address.

(Seventh embodiment)

In the seventh embodiment, the plural inner-product operators generate the pre-synthesis beamforming outputs respectively for the plural adaptive weights for each active channel, and the output combiner 500 acquires from the storing unit (temporary storage 800) and plural inner-product operators, the pre-synthesis beamforming outputs generated for different active channels and sums the outputs, so as to generate the beamforming output.

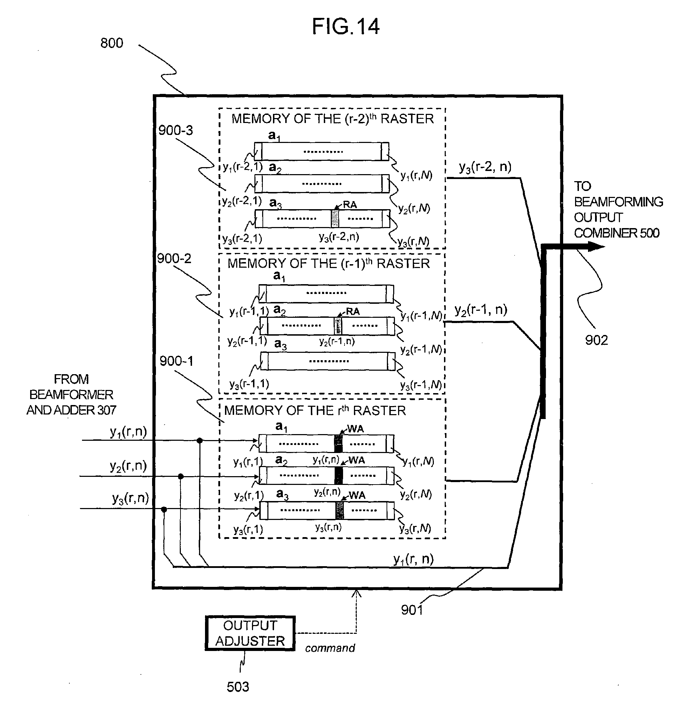

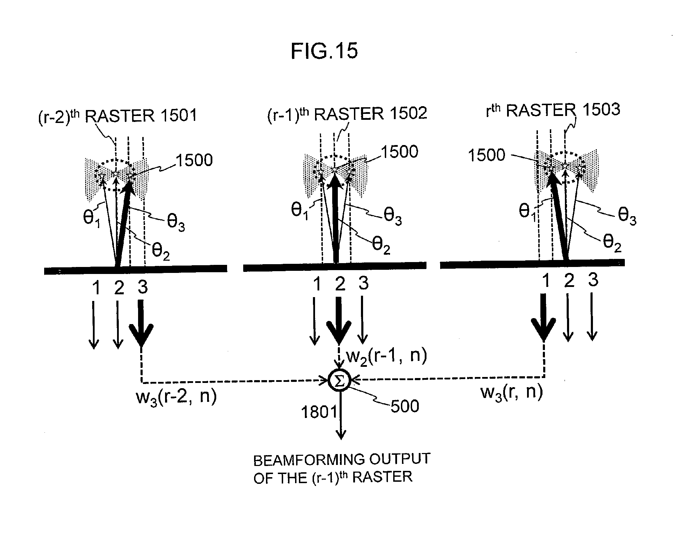

With reference to FIG. 14 and FIG. 15, the ultrasound imaging apparatus according to the seventh embodiment of the present invention will be specifically explained. The configuration being the same as that of the sixth embodiment will not be explained tediously. As shown in FIG. 14, the ultrasound imaging apparatus of the seventh embodiment is provided with the memories 900-1, 900-2, and 900-3 within the temporary storage 800 for storing the beamforming outputs (post-beamforming data) $y_1(n)$ to $y_P(n)$ in association with P steering vectors for the three rasters. The structure of each memory 900-1, 900-2, and 900-3 is the same as the memory 900 of the sixth embodiment. With this configuration, it is possible to store the post-beamforming data in association with three rasters. By way of example, the beamforming data $y_1(r, n)$, $y_2(r, n)$ ... $y_p(r, n)$ at the snapshot time n of the $r^{th}$ raster, the beamforming data $y_1(r-1, n)$, $y_2(r-1, n) \cdots y_p(r-1, n)$ at the snapshot time n of the $(r-1)^{th}$ raster, and the beamforming data $y_1(r-2, n)$, $y_2(r-2, n)$ ... $y_p(r-2, n)$ at the snapshot time n of the $(r-2)^{th}$ raster, are respectively stored in the memories 900-1, 900-2, and 900-3. Those data items multiplied by N are stored for each raster, N being the number of snapshots in the time direction.

The current post-beamforming data items $y_1(r, n)$, $y_2(r, n)$, and $y_3(r, n)$ at the snapshot time n are written into the write address WA as shown in the figure, and simultaneously those data items are also outputted to the output combiner 500 via the bypass line 901.

In FIG. 14, since the number of the steering vectors is assumed as P = 3, for ease of illustration, the post-beamforming data at one snapshot time n corresponds to three data items $y_1(n)$ to $y_3(n)$ for each raster. However, the present embodiment is not limited to the example P = 3, but the number of the steering vectors is any number at least 2. Other configurations are the same as those of the sixth embodiment, and tedious explanations will not be provided.

According to the present embodiment, the post-beamforming data items $y_1(r,1)$, $y_2(r,1)$ ... $y_p(r,1)$ to $y_1(r,N)$, $y_2(r,N)$ ... $y_p(r, N)$ of the current scanning raster (the $r^{th}$ raster) at the snapshot time 1 to N can be held in the memory 900-1, and further, the post-beamforming data items of the $(r-1)^{th}$ raster and the $(r-2)^{th}$ raster are also stored respectively in the memory 900-2 and in the memory 900-3. Therefore, the post-beamforming data items, relating to various rasters, snapshot times, and steering vectors, are respectively stored in the memories 900-1 to 900-3. Accordingly, at least one desired post-beamforming data item is selected from those post-beamforming data items, and outputted to the output combiner 500.

By way of example, as shown in FIG. 15, when the receive focus 1500 of P = 2 ($\theta_2 = 0°$) of the $(r-1)^{th}$ raster, the point 1500 for collecting data in the steering vector of P = 1 ($\theta_1$) in the $r^{th}$ raster, and the point 1500 for collecting data in the steering vector of P = 3 ($\theta_3$) in the $(r-2)^{th}$ raster are overlapping one another, following data items are allowed to be outputted to the output bus 1007 in the direction of the output combiner 500, as shown in FIG. 14 and FIG. 15; the current post-beamforming data $y_1(r, n)$ at the time n at a certain steering angle $\theta_1$ (P = 1) is outputted from the $r^{th}$ raster via the bypass line 901, the data $y_2(r-1, n)$ at the time n, at the steering angle $\theta_2$ (P = 2) is outputted from the $(r-1)^{th}$ raster, and the data $y_3(r-2, n)$ at the time n, at the steering angle $\theta_3$ is outputted from the $(r-2)^{th}$ raster. In this situation, the formula (23) expresses the combining process performed by the output combiner 500.

[Formula 23]

$$y_{\text{sum}}(n) = \sum_{p=1}^{3} y_p(n) = \frac{1}{3}\left\{ y_1(r,n) + y_2(r-1,n) + y_3(r-2,n) \right\} \quad \cdots \quad (2\ 3)$$

According to this process, post-beamforming data items acquired from plural rasters are combined with regard to an identical focus 1500, and compared to the case of using the post-beamforming data obtained from one steering vector in a single raster, it is possible to achieve higher resolution of the point image, and to reduce noise caused by combining plural rasters.

It is to be noted that the post-beamforming data items stores in the memories 900-1, 900-2, and 900-3 are overwritten every time the raster is updated, and the latest post-beamforming data items for the three rasters are constantly stored in the memories 900-1, 900-2, and 900-3, respectively.

Further in the seventh embodiment, the output adjuster 503 may be provided. In this case, the output adjuster 503 transfers to the temporary storage 800, a signal to control the data address to be outputted and its timing to the output combiner 500.

(Eighth embodiment)

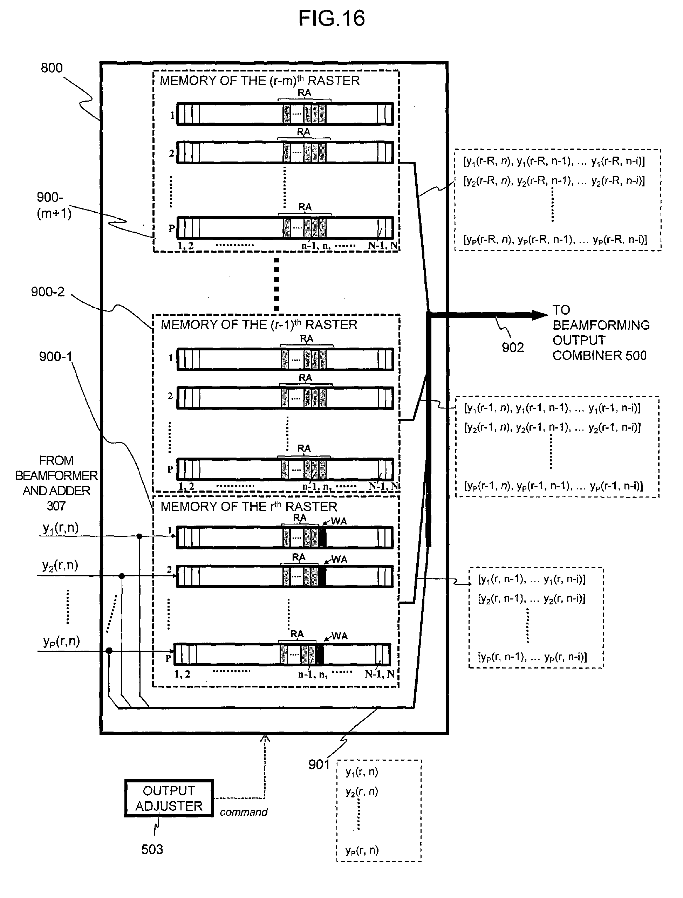

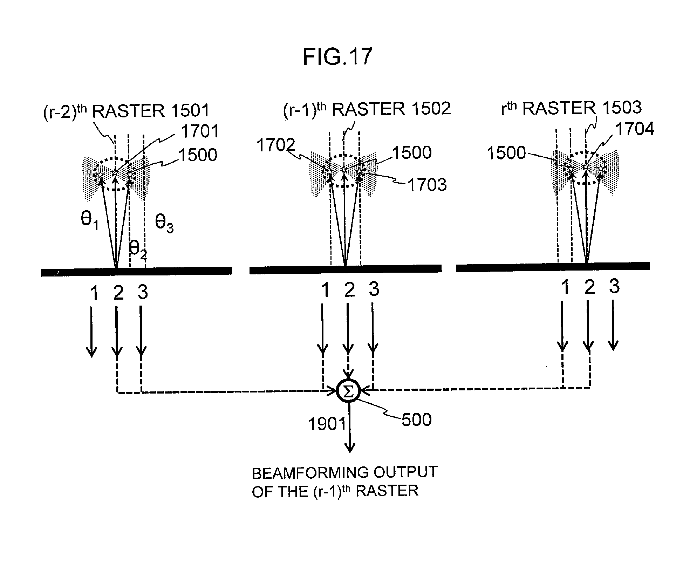

With reference to FIG. 16 and FIG. 17, the ultrasound imaging apparatus according to the eighth embodiment of the present invention will be explained. As illustrated in FIG. 16, in the ultrasound imaging apparatus of the eighth embodiment, there are arranged (m + 1) memories 900-1, 900-2 ... 900-(m+1) within the temporary storage 800. Each of the memories 900-1 to 900-(m+1) has the same configuration as the memory 900-1 of the seventh embodiment. With this configuration, at least one of any combination is allowed to be selected from all the combinations of the post-beamforming data with regard to the three-dimensions; the plural steering angle directions 1 to P in an identical raster, the snapshot times 1 to N, and the raster shifting directions 1 to (m + 1), and the selected combination is outputted to the output combiner 500 for synthesis. In other words, the outputs 1106, 1107, and 1108 to be combined are selected from P $\times$ N $\times$ (m+1) post-beamforming data items, and outputted to the output combiner 500 so as to be combined. It is to be noted that the configuration being the same as the seventh embodiment will not be explained tediously.

Therefore, as shown FIG. 17, it is possible to select and combine the beamforming outputs not only within an identical raster, but also the beamforming outputs at any angle from adjacent rasters. In other words, similar to the seventh embodiment, the beamforming data of the identical focus 1500 is obtained from different rasters 1501, 1502, and 1503, and those data items are combined, thereby acquiring the data of the focus 1500 repeatedly, and enhancing the precision of data. Further in the eighth embodiment, unlike the seventh embodiment, the post-beamforming data items as to the points 1701 to 1704 around the focus 1500 are also selected and outputted, and then the output combiner 500 is allowed to combine those data items. As illustrated in FIG. 5 (c), it is further possible to combine the information (post-beamforming data) of the point spread function 2204 around the focus 1500.

In other words, as seen from FIG. 5(c), not only the data of one focus 1500 can be acquired repeatedly, but also the signals of the object scattered in the elliptic region 2205 around the focus 1500 can be obtained. Therefore, with reduction of failing to pick up signals, correlative noise may also be reduced.

As shown in FIG. 5(d), it is further possible to combine the post-beamforming data items on the distant two points 1711 and 1712 in the elliptic region 2205 where the signals are scattered, and the post-beamforming data of the point spread function 2204 around those two points. With this configuration, even though the overlaps of the point spread function 2204 is small, most of the elliptic region 2205 is allowed to be covered by the point spread function 2204, and thus this reduces failing to pick up signals within the elliptic region 2205 together with reducing the correlative noise.

According to the present embodiment, though not clearly illustrated in FIG. 17, any point in the time direction may also be selected for combine the post-beamforming data at that time, and this allows collection of the data items 1701 and 1702 scattered inhomogeneously in the time direction, and it is also possible to compensate for the deterioration of the image quality of the ultrasound image in the time direction (in the ultrasound propagating direction).

Also in the present embodiment, the output adjuster 503 may be provided, so as to control the data address to be outputted and its timing to the output combiner 500.

In the seventh and the eighth embodiments, information in the adjacent rasters is used, and thus at the clock time of the $r^{th}$ raster being currently scanning, the $(r-1)^{th}$ combined data is transferred to the image processor 109. Therefore, there is a certain delay for the timing of image processing.

As illustrated in FIG. 5(e), since the overlaps of the point spread function 2204 in FIG. 5(b) and the overlaps of the point spread function 2204 in FIG. 5(c) are performed simultaneously, this allows further reduction of failing to pick up the signals within the elliptic region 2205. In addition, the overlaps of the point spread function 2204 in FIG. 5(e) and the overlaps of the point spread function 2204 in FIG. 5(d) are performed simultaneously, thereby allowing further reduction of failing to pick up the signals within the elliptic region 2205, and also reducing the correlative noise more.

(Ninth embodiment)

In the ninth embodiment, an explanation will be made as to the plural elements 106 that are in the form of a two-dimensional array, i.e., being arranged two-dimensionally. The peripheral information operator 205 generates pre-synthesis beamforming outputs as to plural points being set two-dimensionally. The storing unit (temporary storage) 800 stores those pre-synthesis beamforming outputs.

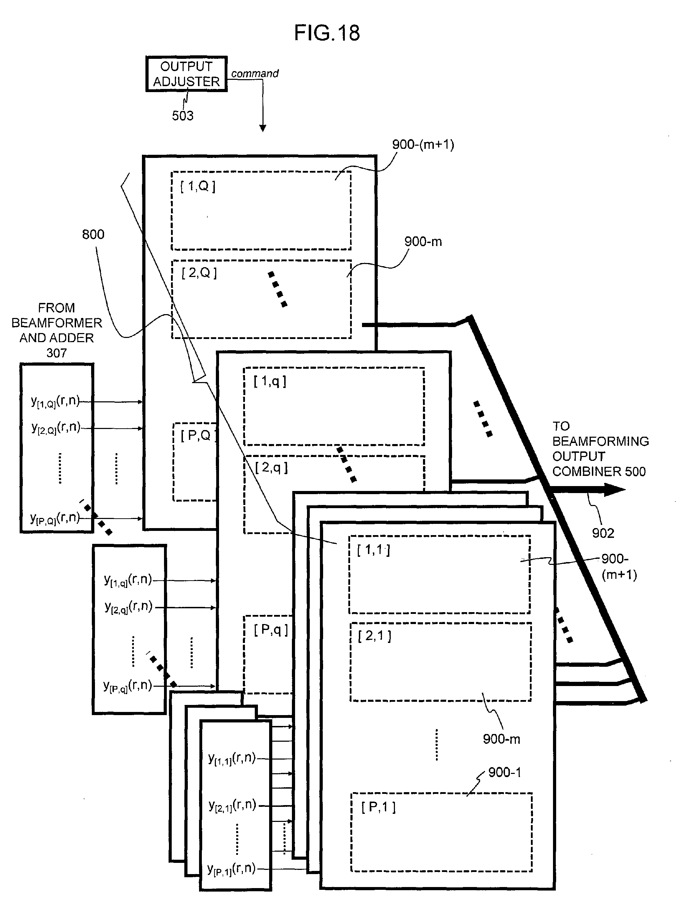

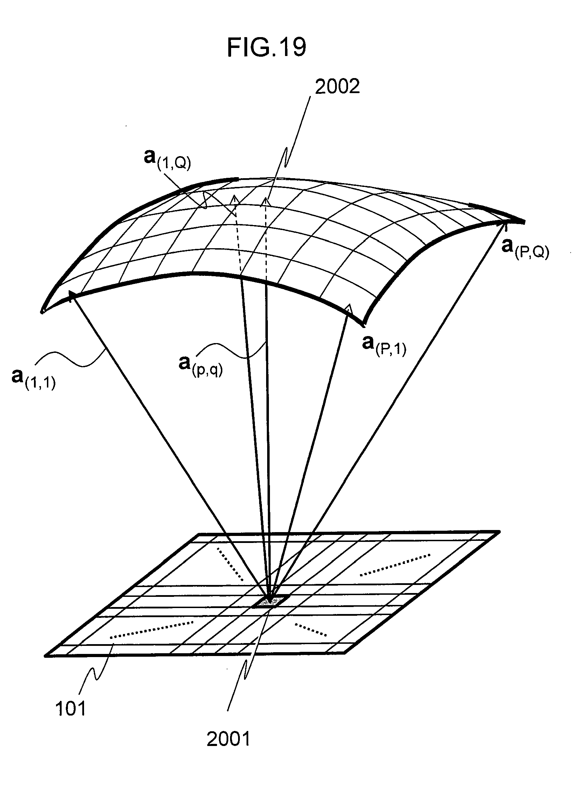

With reference to FIG. 18 and FIG. 19, the ultrasound imaging apparatus according to the ninth embodiment of the present invention will be explained specifically. As shown in FIG. 18, the ultrasound imaging apparatus of the ninth embodiment has a configuration that Q temporary storages 800 of the eighth embodiment as shown in FIG. 16 are arranged in parallel. A probe in which the elements 106 are two dimensionally arranged is employed as the probe 101. By way of example, a two-dimensional array probe, a two-dimensional array convex probe, a probe with a bundle of short-axes, or the like, may be used. It is alternatively possible to use a probe (mechanical scanning 3D probe) in which an extra dimension is added every time one-dimensional array is moved mechanically, so as to acquire information similar to that of the two-dimensional array. The configuration similar to the eighth embodiment will not be explained tediously.

The adaptive weight operator 301 varies the steering angle being calculated, in the two-dimensional direction where the elements 106 of the probe 101 are arranged, and obtains adaptive weight vectors as to $P \times Q$ steering angles. Within the Q temporary storages 800, the post-beamforming data items obtained as to the adaptive weight vectors for the respective steering vectors are stored at every snapshot time. With this configuration, it is possible to collect the peripheral information (post-beamforming data) three-dimensionally.

In the present embodiment, the steering vectors $a_p$ expressed by the aforementioned formula (4) are extended in the two-dimensional direction, and the present invention allows the steering vector direction to be selected, in the form of any combination of $(\theta_p, \varphi_q)$, particularly using $1 \leq p \leq P$ and $1 \leq q \leq Q$ in the two dimensional angles. The formula (24) expresses the steering vector $a_{(p, q)}$ for this case.

[Formula 24]

$$\mathbf{a}_{(p,q)} = \left[ \exp\left\{ \psi_1\left(\theta_p, \phi_q, f_{(p,q)}\right)\right\}, \exp\left\{ \psi_2\left(\theta_p, \phi_q, f_{(p,q)}\right)\right\}, \ldots, \exp\left\{ \psi_K\left(\theta_p, \phi_q, f_{(p,q)}\right)\right\} \right]$$

$$\cdots \ (2 4)$$

FIG. 19 illustrates the operations of the signal processing for the ninth embodiment. Upon viewing the test subject 100 from the element 2001 at the center point on the two-dimensional array probe 101, it is possible to collect information of the object on a sphere assuming as the radius, a line segment connecting the receive focus 2002 and the element center 2001. By way of example, in the formula (24), $P \times Q$ steering vectors $a_{(p, q)}$ associated with the angles $(\theta_p, \varphi_q)$ in the two-dimensional direction may be set on the sphere.

(Tenth embodiment)

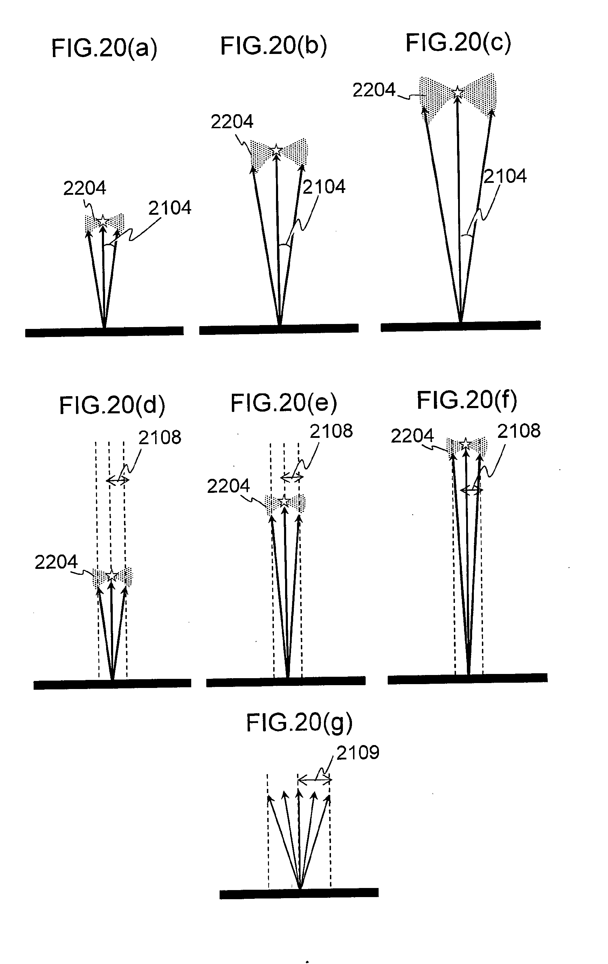

In the tenth embodiment, an explanation will be provided as to the adjuster 501 that adjusts at least either one of the number of the steering vectors and the directions thereof. FIG. 20 illustrates an example in which the adjuster 501 of FIG. 11 sets the steering vectors. The steering vector direction may be determined by a predetermined fixed angle, or the direction may be changed according to the control by the adjuster 501.

FIG. 20(a) to FIG. 20(f) illustrate two examples of the steering angle, as the embodiment thereof. FIG. 20 (a) to FIG. 20 (c) illustrate that the steering angle 2104 is fixed. In the order from FIG. 20(a) to FIG. 20(c), the depth of the imaging target point (data collection point) becomes deeper. Since the steering angle 2104 is fixed, even though the steering vectors are kept the same, the deeper is the imaging target point, the range (the point spread function 2204 (FIG. 5(a)) becomes larger, the range allowing the signals to be collected around the imaging target point.

FIG. 20(d) to FIG. 20(f) illustrate an example that the steering angle becomes smaller, as the imaging target becomes deeper, and the horizontal distance 2108 (spread of imaging target point) is made constant, the horizontal distance indicating the distance from the receive focus to the point expected by the steering vector. In other words, in one raster, the steering angle is made to vary for each depth (snapshot time n) of the imaging target point, thereby rendering the spread of the imaging target point (point spread function 2204) to be constant irrespective of the depth of the imaging target.

In addition, as shown in FIG. 20 (g), the adjuster 501 is allowed to set the number of the steering vectors within the steering angle 2109, or the angle of adjacent steering vectors.

As for a combination of the steering angle and the number of the steering vectors, the adjuster 501 stores data in advance in such a manner as storing plural types of combinations of the steering angle and the number of the vectors, and depending on the focal position (the position of the imaging target point), the imaging portion of the test subject 100, and the imaging sequence, the adjuster 501 selects a suitable combination of the steering angle and the number of the vectors, so as to set the selected combination in the angle adjuster 502 and in the output adjuster 503. It is alternatively possible that the operator selects via the console 110, a combination of the steering angle and the number of vectors, or the operator inputs in the adjuster 501 via the console 110, any steering angle and any number of the vectors.

(Eleventh embodiment)

In the eleventh embodiment, there is further provided a memory configured to store in advance, a distribution of the spread angles of the steering vectors directed to plural points, in association with the position of the receive focus within the image that is generated by the image processor. The adjuster uses the steering vectors having the spread angles read out from the memory, in association with the position of the receive focus, so as to obtain the adaptive weights. The spread angle of the steering vector stored in the memory is configured to be small for the receive focus being close to the send focus upon transmitting ultrasound wave signals to the test subject, whereas it is configured to be large for the receive focus being distant from the send focus. In addition, the spread angle of the steering vector is configured to be smaller at the edge part of the image, compared to the center part of the image.

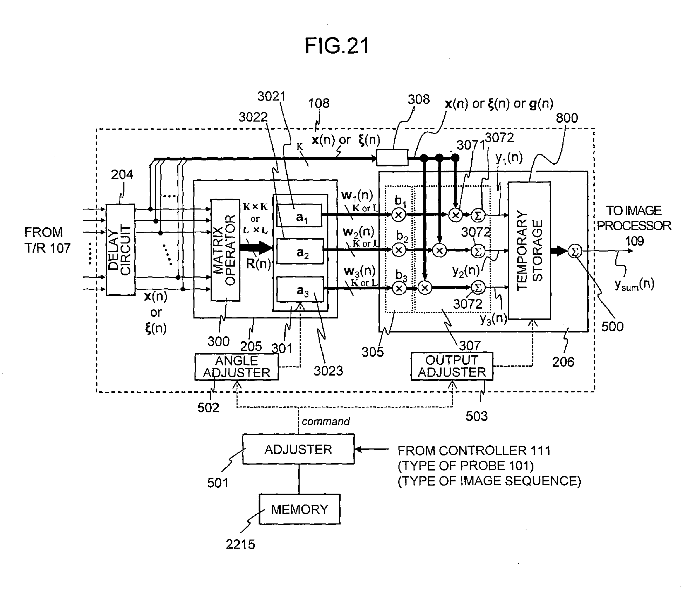

With reference to FIG. 21 and FIG. 22, the ultrasound imaging apparatus of the eleventh embodiment will be explained specifically. In the present embodiment, the adjuster 501 applies the steering angles different for each imaging target point within one image, depending on the type of the ultrasound probe 101, imaging conditions, and a type of the imaging sequence, and then an image is generated. By way of example, various sets of steering angles are applied to generate an image, in association with the probe, such as a linear probe, a convex probe, a sector probe, a two-dimensional array probe, a mechanical 3D imaging probe, and the like. In addition, various sets of steering angles are applied to generate an image, for example, the steering angles being different depending on the imaging conditions such as the send focus, receive focus, send/receive frequency, frame rate, parallel beamforming count, tissue harmonics, and contrast, and the imaging sequence.

In general, the focus upon sending is set on at least one fixed position when one ultrasound image is taken, and the receive beamformer 104 varies the receive focus, thereby obtaining an image for each imaging target point within the imaging area. Therefore, the focus 2210 upon sending is determined at a position within the image. On the other hand, as explained with reference to FIG. 5(a), signals of the object spread in the elliptic region 2205 centering on the receive focus, due to the reflection on the receive focus, and the size of the region varies depending on the depth (time) of the receive focus. As illustrated in Fig. 22(a), the size do the elliptic region 2205 is smaller as it is closer to the send focus 2210, and it becomes larger with distance from the send focus 2210. Therefore, when the beam forming is performed using the steering vectors with a fixed steering angle (spread angle) 2211, there may occur a mismatch between the elliptic region 2205 indicating the spreading of the signals of the object, and the size of the point spread function 2204 allowing signal acquisition, determined by the spread angles of the steering vectors.

In the periphery of the first raster and the last raster in the scanning direction among the rasters upon receiving, that is, on the image edge parts, a part of the point spread function 2204 where signals are allowed to be acquired, being determined by the spread angles of the steering vectors, runs off the image as shown in FIG. 22(a). Therefore, signal strength acquired on the image edge is lowered, compared to the image center.

The aforementioned mismatch and extending off the image of the point spread function 2204 may result in that intensity unevenness shows up in a B-mode image. For example, as illustrated in FIG. 22(b), the structure around the send focus 2210 is depicted with homogeneous intensity, but in a shallow part, a deep part, and the image edge part, the image intensity may be deteriorated and unevenness may occur, due to the mismatch between the elliptic region 2205 indicating the spread of the signals of the object and the point spread function 2204, and extending off the image of the point spread function 2204.

In the eleventh embodiment, in order to reduce the intensity unevenness in the depth direction, the steering spread angle is varied in accordance with the signal spreading region of the object, so that the point spread function 2204 upon beamforming matches the elliptic region 2205. In the image edge part, a steering angle is used which restrains spreading of the point spread function 2204 determined by the spread angles of the steering vectors, so that the point spread function may not expand unnecessarily out of the imaging target. By way of example, beamforming according to the following methods may be performed at the image edge part; a method of reducing the steering angle gradually, as the object becomes deeper, a method of preparing a fixed small steering angle, a method of using an output of normal adaptive beamforming (an output using the steering vectors of $\theta_p = 0°$) for the edge part, without performing synthesis of the steering angles, and the like.

In order to implement the configuration above, the adjuster 501 is provided in the memory 2215 as shown in FIG. 21, in the eleventh embodiment. The memory 2215 stores in advance, data in the form of table, or the like, defining a distribution of the spread angles of the steering vectors (steering angles), with regard to the scanning direction and the depth (time) direction of the raster. The distribution of the steering angles is defined so as to enhance the image intensity and reduce the unevenness, considering that the variation of the size of the elliptic region 2205 indicating the spread of the signals of the object, depends on the type of the probe 101 and the imaging sequence (a focus upon sending, and the like). The distribution of the steering angles is prepared for each combination of the type of the probe 101 and the imaging sequence. The distribution of the steering angles for enhancing the image intensity and reducing unevenness may be obtained by calculations (including a computer simulation), experiments, and the like, performed in advance.

The adjuster 501 receives from the controller 111, information regarding the type of the probe 101 and the imaging sequence, and reads out from the memory 2215, data defining the distribution of the spread angles of the steering vectors in association with the information. According to the data being read out, the adjuster 501 sets the spread angles (steering angles) of the steering vectors in the angle adjuster 502 and in the output adjuster 503 in the receive beamformer 108, for each scanning direction and depth (time) direction of the raster.

Accordingly, it is possible to allow the point spread function 2204 upon beamforming to coincide with the elliptic region 2205 indicating the spread of the signals of the object. Further in the image edge part, the point spread function 2204 is controlled not to expand unnecessarily out of the imaging target. Therefore, as shown in FIG. 22(d), it is possible to create an image not including any intensity unevenness, and it is effective for enhancing the image quality.

In the eleventh embodiment, the configuration of the receive beamformer 108 is not limited to the configuration of FIG. 21, and the receive beamformer 108 performing the receive beamforming of the first to the ninth embodiment may be employed.

In this example here, the case where the send focus is fixed to one or plural points is explained. If a dynamic focus for sending is used so as to change the send focus in the depth direction, there may occur unevenness in the elliptic region 2205 indicating the spread of signals of the object. Therefore, the tenth embodiment is applicable.

FIG. 22(a) to FIG. 22(d) illustrate an image obtained by using the convex probe, but the tenth embodiment may be applicable to any other type of probe such as a linear probe, a sector probe, a 2D array probe, a mechanical 3D probe, and any other raster scanning method.

(Twelfth embodiment)

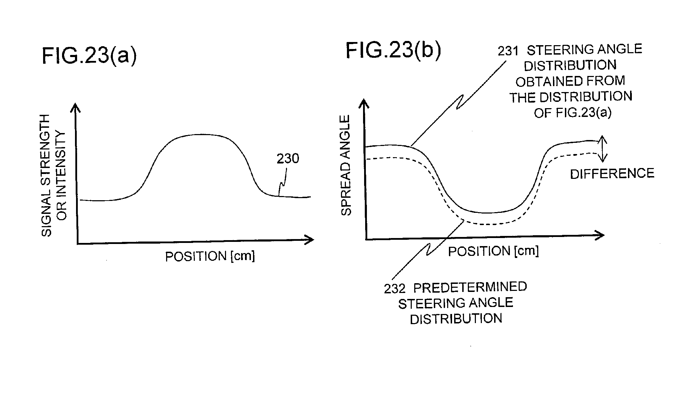

With reference to FIG. 23 (a) and FIG. 23(b), the ultrasound diagnostic apparatus of the twelfth embodiment will be explained. The eleventh embodiment is directed to the configuration that determines in advance the distribution of the steering angles, but in the twelfth embodiment, it is obtained from the distribution of signal strength or the distribution of intensity in the B-mode image. The receive beamforming is performed by using thus obtained distribution of the steering angles. In other words, the adjuster converts the distribution of the intensity or the signal strength of the image, into a distribution of the spread angles of steering vectors, through the use of a predetermined function, the image having been obtained with the setting of steering vectors using the distribution of the spread angles of the steering vectors being stored in the memory. A difference between the distribution of the spread angles of the steering vectors being stored in the memory and the distribution of the spread angles of the steering vectors obtained through the use of the function, is used to correct either the distribution of the spread angles of the steering vectors being stored in the memory, or the distribution of signal strength or the intensity distribution.

Specifically, the beamforming of any of the first to the ninth embodiments is performed initially, with the use of the fixed steering angle as shown in FIG. 22(a), and the B-mode image as shown in FIG. 22 (b) is obtained. With regard to the B-mode image, the adjuster 501 obtains the distribution of intensity or the distribution of the post-beamforming signal strength 230 in the imaging target, as illustrated in FIG. 23(a).

The intensity distribution or the signal strength distribution 230 being obtained is fitted to a predetermined function for converting the distribution of the intensity or the signal strength into the steering angle distribution, so as to obtain the steering angle distribution 231 as shown in FIG. 23(b). Then, the receive beamforming of the eleventh embodiment is performed as shown in FIG. 22 (c), thereby actively improving the intensity unevenness in the image.

The function for converting the distribution of the intensity or signal strength into the steering angle distribution is obtained in advance by experiments, or computations (including a computer simulation), and the memory 2215, or the like, stores the function in the form of database like a table, or in the form of mathematical formula.

As described in the eleventh embodiment, by using the distribution of the spread angles (steering angles) of the steering vectors as stored in the memory 2215, the receive beamforming is performed as shown in FIG. 22(c), and as to thus obtained B-mode image of FIG. 22(d), the intensity distribution or the post-beamforming signal strength distribution 230 within the imaging target may be obtained as illustrated in FIG. 23(a). The obtained intensity distribution of the signal strength distribution is converted into the steering angle distribution 231 through the use of the aforementioned function.

The obtained steering angle distribution 231 is compared with the steering angle distribution 232 used for generating the B-mode image of FIG. 22(d), and a difference therebetween is obtained and the difference is reflected on the beamforming process. By way of example, it is possible to employ a method that modifies the steering angle distribution stored in the memory 2215 in accordance with the difference, and performs the receive beamforming again with the use of the modified distribution of steering angles, or a method that modifies the intensity distribution of the signal strength distribution of the B-mode image, in accordance with the obtained difference.

With the configuration above, it is possible to perform modification with the feedback of the steering angle distribution 232, or the distribution of intensity or signal strength 230 of the image.

As explained in the present embodiment, the steering angle distribution may be obtained actively, or modified with the feedback operation. This process is applicable not only to the intensity unevenness when the adaptive beamformer is utilized, but also the intensity unevenness due to energy inhomogeneity of acoustic signals that occurs also in the beamforming according to a conventional DAS (Delay And Sum: delay addition process). Therefore, this may contribute to enhancement of an ultrasound B-mode image inherently.

(Console of the ultrasound diagnostic apparatus)

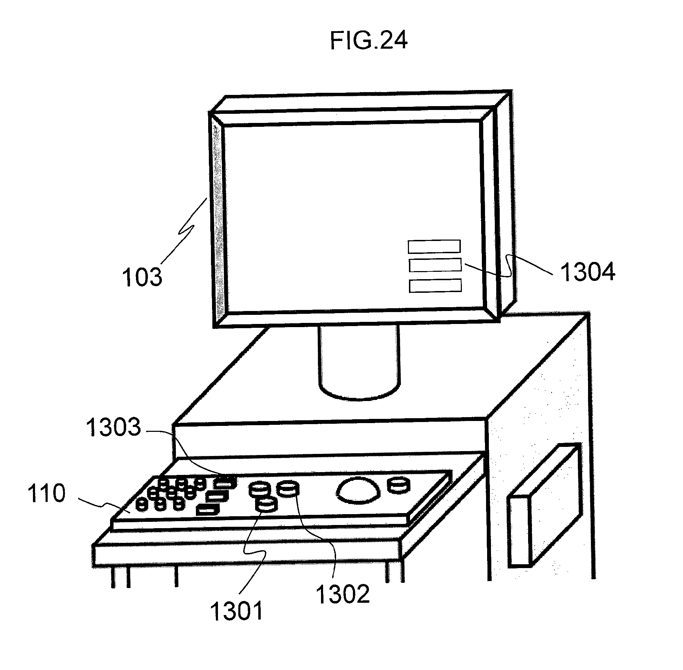

FIG. 24 illustrates one example of the console 110 of the present invention. In order to implement the aforementioned embodiments, there are arranged on the console 110 of the ultrasound diagnostic apparatus, scaled knobs 1301, 1302, and the like, as the operating portion configured to change the number of the steering vectors and the density thereof, and the number of the beamforming results to be combined, in the angle direction, in the time direction, and in the raster direction, and the like. In addition, switches 1303 are provided on the console 110, as the operating portion configured to switch the mode of the steering angle explained in each of the aforementioned embodiments, and to switch between combining the beamforming results and not combining the beamforming results. With the configuration above, the operator is allowed to change various parameters for the beamforming and synthesizing process, while viewing the actual ultrasound image, and conduct diagnosis under conditions being optimum for each test subject 100. Furthermore, the set values may be displayed in a part of the display area 1304 on the monitor 103.

A mode-switching part in the console may perform switching, in association with the various types of the probe, imaging conditions, and imaging sequences. For example, the switching part may switch to the mode that applies a set of various steering angles, being different depending on the probe types, such as a linear probe, a convex probe, a sector probe, a two-dimensional array probe, and a mechanical 3D imaging probe, so as to generate an image. Alternatively, for example, the switching part may switch to the mode that applies a set of various steering angles, being different depending on the imaging conditions, such as the send focus and receive focus, send/receive frequency, frame rate, parallel beamforming count, tissue harmonics, and contrast, and the imaging sequence, so as to generate an image.

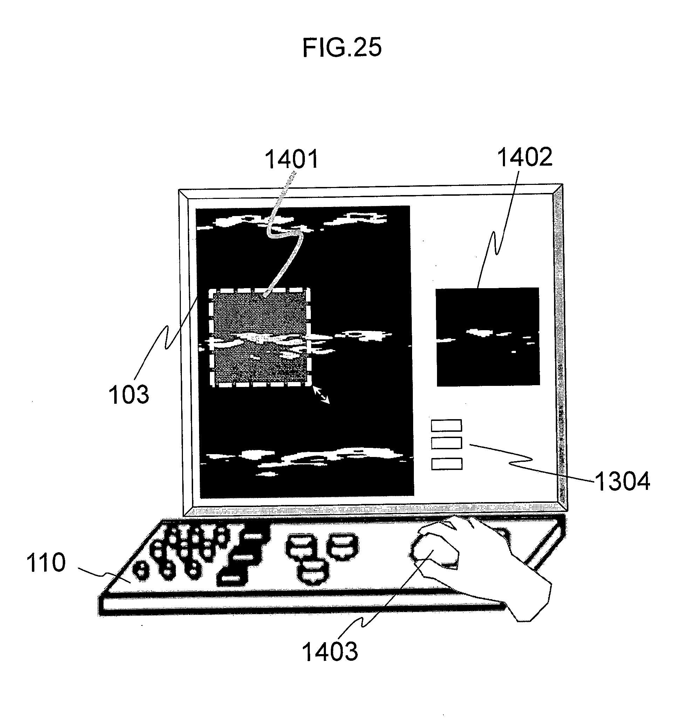

FIG. 25 is a perspective view illustrating another specific example of the console 110 and the monitor 103 of the ultrasound diagnostic apparatus according to the present invention. In the configuration of FIG. 25, there are prepared a manually handling operating portion 1403 (e.g., a mouse) for an operator to set a specific ROI (Region of Interest) 1401, with reference to the normal ultrasound image 103 (to which the synthesis operation of the present invention has not been applied). With this configuration, the operator is allowed to generate the image 1402 after the adaptive processing of the present invention is applied thereto, only as to a specific ROI 1401. Furthermore, the image 1402 after the adaptive processing of the present invention is applied may be displayed in a different area on the monitor 103.

It is further possible to configure such that the operator sets the arithmetic parameters respectively of the peripheral information operator 205 and the peripheral information combiner 206, through the use of the operating portion 1403 that is manipulated by the operator. On this occasion, the adjuster 501 as shown in FIG. 11 receives the arithmetic parameters being set via the operating portion 1403, outputs control signals to the angle adjuster 502 and the combined output adjuster 503, and adjust the arithmetic parameters in the adaptive weight operator 301 and the output data in the temporary storage 800.

(Specific examples of the effects of the embodiments)

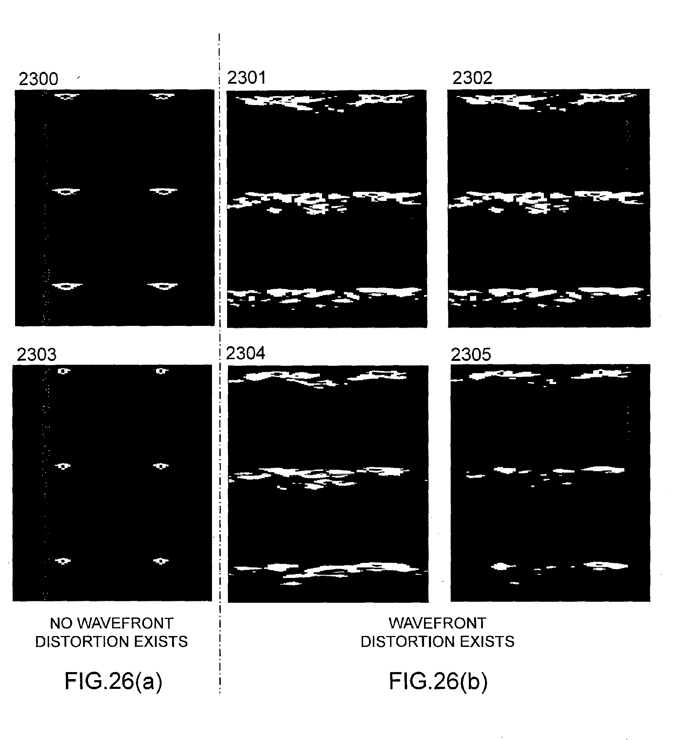

FIG. 26 shows ultrasound images (B-mode images) obtained by simulation for illustrating effects of the embodiments of the present invention. Here, six point scatterers are assumed as the test subject 100, and the ultrasound images thereof are obtained by a computer. FIG. 26(a) illustrates the image 2300 and the image 2303 where no wavefront distortion occurs in the ultrasound beam. The image 2300 is obtained by the receive beamforming according to the conventional delay-and-sum method, and the image 2303 is obtained by the conventional adaptive beamforming. On the other hand, FIG. 26(b) illustrates the images 2301, 2302, 2304, and 2305 where wavefront distortion occurs in the ultrasound beam. The image 2301 is an image obtained by the receive beamforming according to the conventional delay-and-sum method, and the image 2304 is an image obtained by the conventional adaptive beamforming. The image 2302 is an image obtained according to the delay-and-sum method and steering vectors being combined similar to the method of the second or the third embodiment. The image 2305 is an image obtained by performing the adaptive beamforming and combining the steering vectors according to the method of the second or the third embodiment.

When the images 2301 and 2304 including the wavefront distortion are compared with the images 2300 and 2303 not including the wavefront distortion, it is found that even though the same beamforming method is applied, there exist belt-like high-intensity areas around the point scatterers, in the case where the wavefront distortion occurs, and it is difficult to distinguish the point scatterers.

When the image 2301 obtained by the conventional delay-and-sum method is compared with the image 2302 obtained by combining the steering vectors in the conventional delay-and-sum method, it is found that even though the steering vectors are combined in the delay-and-sum method, the belt-like high intensity areas around the scatterers are hardly improved, failing to remove the influence of the wavefront distortion.

On the other hand, when the image 2304 obtained according to the adaptive beamforming is compared with the image 2305 obtained by combining the steering vectors in the adaptive beamforming of the second or the third embodiment, obviously the belt-like high intensity area is decreased in the image 2305, and the six point scatterers are separated and recognizable. Accordingly, it is found that the influence of the wavefront distortion is removed by combining the steering vectors in the adaptive beamforming according to the second or the third embodiment.

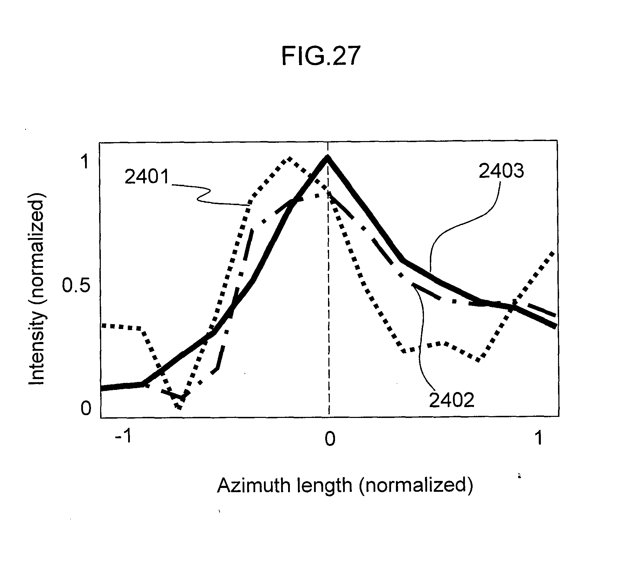

FIG. 27 is a graph showing the intensity distribution of one point scatterer and the surrounding thereof in the images 2301, 2304, and 2305 of FIG. 26. The horizontal axis of the graph in FIG. 27 indicates the azimuth direction of the receive array (scanning direction of the paster), and the vertical axis indicates the intensity of the image. The position where the horizontal axis is just zero corresponds to the position of the point scatterer. The dotted line 2401 in the graph of FIG. 27 indicates the intensity distribution of the image 2301 according to the conventional delay-and-sum method, the dashed-dotted line 2402 indicates the intensity distribution of the image 2304 according to the conventional adaptive beamformer, and the solid line 2403 indicates the intensity distribution of the image 2305 of the adaptive beamformer to which the synthesis of steering vectors in the second or the third embodiment is applied.

As indicated by the dotted line 2401, the intensity peak position in the intensity distribution of the image 2301 according to the delay-and-sum method is displaced from the real position of the object. As indicated by the dashed-dotted line 2402, the intensity peak position in the intensity distribution of the image 2304 according to the conventional adaptive beamformer becomes closer to the real position of the object, but the magnitude of the intensity is lowered. As indicated by the solid line 2403, in the intensity distribution of the image 2305 according to the second or the third embodiment of the present invention, the signal strength is maintained to be equivalent to the intensity distribution by the delay-and-sum method (the dotted line 2401), the peak intensity is located on the real position of the object (the position zero on the horizontal axis), and therefore this ascertains the effect produced by compensating for the wavefront distortion.

As described above, according to the present invention, the adaptive beamformer is provided with the ability of avoiding the failure in picking up information due to sharp beam directivity. In addition, there is provided another ability of canceling and reducing the unnecessary correlative noise from the medium around the focus. Accordingly, the present invention provides the ultrasound imaging apparatus having a robust property against the wavefront distortion, caused by sound-velocity inhomogeneity within a living body, a distribution of scatterers, and body motion influence. The present invention further allows a point image made up of plural perspective angles to become high in resolution, without performing the focus calculation (delay calculation) one by one for each target point in the test subject 100. Therefore, according to the present invention, it is possible to provide the ultrasound imaging apparatus with the adaptive beamformer that needs relatively small processing loads for solving the problems above.

Here is a summary of the configurations of the present invention.

(1) The first embodiment relates to a configuration of the apparatus provided with the peripheral information operator and the peripheral information combiner. In other words, the ultrasound imaging apparatus is provided with plural elements configured to receive ultrasound signals from a test subject, a delay unit configured to delay each of the signals received by the plural elements in association with a predetermined position of the receive focus, and generate post-delay received signals, the peripheral information operator configured to acquire from the post-delay received signals, information items as to plural points on the receive focus and in the region surrounding the receive focus, the peripheral information combiner configured to combine the information items respectively acquired as to the plural points and generate a beamforming output by using the information items being combined, and an image processor configured to generate image data by using the beamforming output.

(2) The fourth embodiment relates to a configuration for performing synthetic aperture in the apparatus as described in the aforementioned item (1). In other words, the fourth embodiment relates to the ultrasound imaging apparatus where the delay unit generates the post-delay received signals respectively as to plural different receive focuses, and the peripheral information operator and the peripheral information combiner acquire the information items as to the plural points on the receive focus and in the region surrounding the receive focus, with respect to each of the plural receive focuses, and generate the beamforming output.

(3) The fourth embodiment also relates to a configuration that there are plural delay units in the apparatus as described in the aforementioned item (2). In other words, there are more than one delay unit, and each of the delay units generates the post-delay received signals for the receive focus that is different for each of the plural delay units. The peripheral information operator and the peripheral information combiner are provided for each of the delay units, acquire the information items from the post-delay received signals generated by the delay unit for the each of the receive focuses, and generate the beamforming output.

(4) The fourth embodiment also relates to a configuration that the synthetic aperture is performed in the apparatus as described in the aforementioned item (2) or in the item (3). In other words, there is further provided an active channel setter configured to set active channels sequentially to the plural elements, at the positions being different in time series, and transfer to the delay unit, the received signals of the elements included in the active channel. On this occasion, the positions of the plural receive focuses as to the received signals in the active channel at a certain point of time partially overlap the positions of the plural receive focuses as to the active channel at a different point of time.

(5) The second embodiment relates to a configuration that the adaptive beamforming is applied, in any of the apparatus in the aforementioned items (1) to (4). In other words, the peripheral information operator performs the adaptive beamforming, thereby obtaining the adaptive weight as the information item.

(6) The second embodiment relates to a configuration that the peripheral information operator in the apparatus as described in the aforementioned item (5) uses the steering vectors being directional vectors connecting a predetermined element among the plural elements and the plural points, so as to obtain the adaptive weights as to the plural points.

(7) The second embodiment relates to a configuration that in the apparatus as described in the aforementioned item (6), the covariance matrix is used to obtain the adaptive weight vectors. In other words, the peripheral information operator includes the matrix operator configured to use the post-delay received signals to generate the covariance matrix, and the weight vector operator configured to obtain the adaptive weight vectors as to the plural points, from the covariance matrix and the steering vectors.

(8) The second embodiment relates to a configuration that in the apparatus as described in any of the aforementioned items from (5) to (7), the adaptive weights are combined. In other words, the peripheral information combiner includes the weight combiner configured to sum the adaptive weights as to the plural points obtained by the peripheral information operator and generate the combined weight, and the inner-product operator configured to perform inner-product operation between the combined weight and the post-delay received signals, and generate the beamforming output.

(9) The second embodiment relates to a configuration that in the apparatus as described in the aforementioned item (8), the adaptive weight is multiplied by the fixed weight. That is, between the peripheral information operator and the weight combiner, there is arranged a fixed apodization multiplier configured to multiply the adaptive weights as to the plural points obtained by the peripheral information operator, respectively by the predetermined fixed weights.

(10) The second embodiment also relates to a configuration that in the apparatus as described in the aforementioned item (8), assigning weights on the post-delay received signals and summing the signals are performed. In other words, the inner-product operator multiplies each of the post-delay received signals by the combined weight, and thereafter, sums the post-delay received signals to generate the beamforming output.

(11) The third embodiment relates to a configuration that in the apparatus as described in any of the aforementioned items from (5) to (7), the received signals as to the plural points are subjected to beamforming using the respective adaptive weights, and then those signals are added. In other words, the peripheral information combiner includes the plural inner-product operators configured to perform inner-product operation between the adaptive weights as to the plural points obtained by the peripheral information operator and the post-delay received signals respectively, and generate the pre-synthesis beamforming outputs for the respective plural adaptive weights, and the output combiner configured to add and combine the pre-synthesis beamforming outputs as to the plural adaptive weights, and generate the beamforming output that is used for generating the image data.

(12) The third embodiment also relates to a configuration that in the apparatus as described in the aforementioned item (11), the plural adaptive weights are multiplied respectively by the fixed weights. That is, between the peripheral information operator and the plural inner-product operators, there is arranged the fixed apodization multiplier configured to multiply the adaptive weights as to the plural points obtained by the peripheral information operator, by predetermined fixed weights, respectively.

(13) The third embodiment also relates to a configuration that in the apparatus as described in the aforementioned item (11), the post-delay received signals after the beamforming are multiplied by the fixed weights, respectively. That is, between the plural inner-product operators and the output combiner, there is arranged the fixed apodization multiplier configured to multiply the post-delay received signals after the beamforming as to the plural points, by predetermined fixed weights, respectively.

(13-A) The third embodiment also relates to a configuration that in the apparatus as described in the aforementioned item (11), assigning weights on the post-delay received signals and adding the signals are performed. In other words, the plural inner-product operators are made up of a set of multipliers and plural adders, and the plural multipliers multiply the post-delay received signals by the adaptive weights as to the plural points respectively, and thereafter, in the plural adders, the post-delay received signals are added, thereby generating the pre-synthesis beamforming outputs.

(13-B) The third embodiment also relates to a configuration that in the apparatus as described in the aforementioned item (13-A), multiplication by the fixed weights is performed. That is, between the plural multipliers and the plural adders, there is arranged the fixed apodization multiplier configured to multiply the post-multiplication received signals through the plural multipliers, by the fixed weights being predetermined as to the plural points obtained by the peripheral information operator.

(14) The third embodiment also relates to a configuration that in the apparatus as described in any of the aforementioned items (1) to (13), the beamforming outputs are combined within an identical raster. In other words, there is further provided an active channel setter configured to set active channels sequentially to the plural elements, at the positions being different in time series, and transfer to the delay unit, the received signals of the elements included in the active channels. The peripheral information combiner combines the information items that the peripheral information operator obtains from the received signals in one of the active channels, and generates a final beamforming output that is used by the image processor.

(15) The third embodiment also relates to a configuration that in the apparatus as described in the aforementioned items (11), the beamforming outputs are combined within an identical raster. In other words, there is further provided the active channel setter configured to set active channels sequentially to the plural elements, at the positions being different in time series, and transfer to the delay unit, the received signals of the elements included in the active channels. The peripheral information combiner sums the pre-synthesis beamforming outputs respectively as to the plural adaptive weights that the peripheral information operator obtains from the received signals in one of the active channels, and generates a final beamforming output that is used by the image processor, with respect to each of the active channels.

(16) The fifth embodiment relates to a configuration that in the apparatus as described in the aforementioned item (11), synthesizing is performed by the use of a temporary storage. In other words, between the beamformer and the adder, there is provided the storing unit configured to store the pre-synthesis beamforming outputs generated by the beamformer, as to each of the plural adaptive weights.

(17) The sixth embodiment relates to a configuration that in the apparatus as described in the aforementioned item (16), synthesizing is performed using plural samples in the time direction. That is, the adder acquires from the storing unit and the beamformer, the pre-synthesis beamforming outputs different in the time direction, and sums those pre-synthesis beamforming outputs, so as to generate the beamforming output.

(18) The seventh embodiment and the eighth embodiment relate to a configuration that in the apparatus as described in the aforementioned item (16), synthesizing is performed between plural rasters. In other words, there is further provided the active channel setter configured to set active channels sequentially to the plural elements, at the positions being different in time series, and transfer to the delay unit, the received signals of the elements included in the active channels. The beamformer generates the pre-synthesis beamforming outputs respectively as to the plural adaptive weights, for each of the active channels, the adder acquires from the storing unit and the beamformer, the pre-synthesis beamforming outputs generated for the different active channels and sums the beamforming outputs, so as to generate the beamforming output.

(19) The ninth embodiment relates to a configuration that in the apparatus as described in the aforementioned item (16), the plural elements correspond to a two-dimensional array arranged two-dimensionally, and the peripheral information operator generates the pre-synthesis beamforming outputs as to the plural points set in the two-dimensional direction, and the storing unit stores the pre-synthesis beamforming outputs.

(20) The tenth embodiment relates to a configuration that in the apparatus as described in the aforementioned item (6), there is provided the adjuster configured to adjust at least either one of the number of the steering vectors and the directions of the steering vectors.

(21) The eleventh embodiment relates to a configuration that in the apparatus as described in the aforementioned item (20), setting of the steering angle distribution within an image is performed. In other words, there is provided the memory configured to store in advance a distribution of spread angles of the steering vectors directed to the plural points, in association with the positions of the receive focus within the image that is generated by the image processor, and the adjuster obtains the adaptive weights, by using the steering vectors with the spread angles being read from the memory, in association with the position of the receive focus.

(22) The eleventh embodiment relates to a relation between the steering angle distribution and the send focus in the apparatus as described in the aforementioned item (21). In other words, the spread angle of the steering vector stored in the memory is set to be small on the receive focus being close to the send focus upon sending the ultrasound signal to the test subject, and the spread angle is set to be large at the receive angle being distant from the send focus.

(23) The eleventh embodiment relates to a relation between the steering angle distribution and the image edge in the apparatus as described in the aforementioned item (21) or item (22). In other words, the spread angle of the steering vector stored in the memory is set to be smaller at the edge portion, compared to the center of the image.

(24) The twelfth embodiment relates to a configuration that in the apparatus as described in any of the aforementioned items (21), (22), and (23), the steering angle distribution within the image is computed from the B-mode image. In other words, the adjuster employs a predetermined function to convert the distribution of the B-mode image intensity or signal strength of the test subject imaged in advance, into the spread angle distribution of the steering vectors being obtained, and uses thus obtained spread angle distribution of the steering vectors.

(25) The twelfth embodiment relates to a configuration that in the apparatus as described in any of the aforementioned items (21), (22), and (23), the steering angle distribution within the image is corrected. In other words, the adjuster uses the predetermined function to convert the distribution of the B-mode image intensity or signal strength, obtained by setting the steering vectors with the use of the spread angle distribution of the steering vectors that is stored in the memory, into the distribution of the spread angle of the steering vectors. With the use of a difference between the spread angle distribution of the steering vectors within the memory, and the spread angle distribution of the steering vectors obtained using the function, the spread angle distribution, the image intensity, or the signal strength of the steering vectors within the memory is corrected.

(26) The apparatus as described above is further provided with the monitor for displaying the image and the operating portion, and at least either of the monitor and the operating portion is provided with an accepting portion for accepting from the operator, a setting of the steering vectors in the adjuster.

Explanation of References

| | |
|---|---|
| 100 | TEST SUBJECT |
| 101 | ULTRASOUND PROBE |
| 102 | ULTRASOUND IMAGING APPARATUS |
| 103 | MONITOR |
| 104 | TRANSMIT BEAMFORMER |
| 106 | ARRAY-LIKE ELEMENTS (ULTRASOUND TRANSDUCER) |
| 107 | TRANSMIT-RECEIVE SEPARATION CIRCUIT (T/R) |
| 108 | RECEIVE BEAMFORMER |
| 109 | IMAGE PROCESSOR |
| 110 | CONSOLE |
| 111 | CONTROLLER |
| 201, 202, 203 | ACTIVE CHANNEL |
| 204 | DELAY CIRCUIT |
| 205 | PERIPHERAL INFORMATION OPERATOR |
| 206 | PERIPHERAL INFORMATION COMBINER |
| 207 | BYPASS LINE OF POST-DELAY RECEIVED DATA |
| 300 | MATRIX OPERATOR |
| 301 | ADAPTIVE WEIGHT OPERATOR |
| 305 | FIXED APODIZATION MULTIPLIER |
| 306 | WEIGHT COMBINER |
| 307 | INNER-PRODUCT OPERATOR |
| 3071 | MULTIPLIER |
| 3072 | ADDER |
| 308 | DIMENSIONAL COMPRESSOR |
| 401, 402, 403 | ONE POINT IN SPACE |
| 404 | PRIOR-DELAY RECEIVED SIGNAL |
| 405, 406, 407 | CONCAVE FOCUS |
| 408, 409, 410 | RECEIVED SIGNAL (POST-DELAY RECEIVED DATA) WITH ALIGNED WAVEFRONT |
| 500 | OUTPUT COMBINER |
| 501 | ADJUSTER |
| 502 | ANGLE ADJUSTER |
| 503 | OUTPUT ADJUSTER |
| 601, 603 | DIFFERENT POINTS ON THE AXIS NEAR THE CENTRAL AXIS |
| 602 | POINT ON THE CENTRAL AXIS |
| 604, 605, 606 | DELAY CONCAVE FOCUS |
| 800 | TEMPORARY STORAGE |
| 900 | MEMORY PART |
| 901 | BYPASS LINE OF POST-BEAMFORMING DATA |
| 902 | OUTPUT BUS |
| 9001, 9002, 9003 | MEMORY AREA HAVING N ADDRESSES |
| 1301, 1302 | SCALED KNOB |
| 1303 | SWITCH PART |

| 1304 | A PART OF DISPLAY AREA |
| 1401 | REGION OF INTEREST (ROI) |
| 1402 | IMAGE SUBJECTED TO ADAPTIVE PROCESS |
| 1403 | MANUALLY OPERATING PORTION |
| 1501, 1502, 1503 | RASTER |
| 1504, 1505, 1506 | BEAMFORMING OUTPUT |
| 1600 | CENTRAL AXIS OF ACTIVE CHANNEL |
| 1602, 1604, 1606 | STEERING ANGLE |
| 2071, 2072,2073 DATA | BYPASS LINE OF POST-DELAY RECEIVED |
| 2201 | RECEIVE FOCUS |
| 2202 | POINT SPREAD FUNCTION OF DELAY-AND-SUM METHOD |
| 2204 | POINT SPREAD FUNCTION OF ADAPTIVE BEAMFORMER |
| 2205 | ELLIPTIC REGION WHERE FOCUS DATA EXISTS |
| 3021, 3022, 3023 | WEIGHT VECTOR OPERATOR |

**Claims**

1. An ultrasound imaging apparatus comprising,
   plural elements configured to receive ultrasound signals from a test subject,
   a delay unit configured to delay each of the signals received by the plural elements in association with a position of a receive focus, and generate post-delay received signals,
   a peripheral information operator configured to acquire from the post-delay received signals, information items as to plural points within a region including the receive focus and a region surrounding the receive focus,
   a peripheral information combiner configured to combine the information items respectively acquired as to the plural points, and generate a beamforming output by using the information items being combined, and
   an image processor configured to generate image data by using the beamforming output.

2. The ultrasound imaging apparatus according to claim 1, wherein,
   the delay unit generates the post-delay received signals respectively as to plural different receive focuses, and
   the peripheral information operator and the peripheral information combiner acquire the information items as to the plural points within a region including the receive focus and the region surrounding the receive focus, with respect to each of the plural receive focuses, and generate the beamforming output.

3. The ultrasound imaging apparatus according to claim 2, further comprising,
   an active channel setter configured to set active channels sequentially to the plural elements, at the positions being different in time series, and transfer to the delay unit, the received signals of the elements included in the active channel, wherein,
   positions of the plural receive focuses as to the received signals in the active channel at a certain point of time, partially overlap the positions of the plural receive focuses as to the active channel at a different point of time.

4. The ultrasound imaging apparatus according to any one of claims 1 to 3, wherein,
   the peripheral information operator performs adaptive beamforming, thereby obtaining an adaptive weight as the information item.

5. The ultrasound imaging apparatus according to claim 4, wherein,
   the peripheral information operator uses steering vectors being directional vectors connecting a predetermined element among the plural elements and the plural points, so as to obtain the adaptive weights as to the plural points.

6. The ultrasound imaging apparatus according to either of claim 4 and claim 5, wherein,
   the peripheral information combiner comprises,
   a weight combiner configured to sum the adaptive weights as to the plural points obtained by the peripheral information operator and generate a combined weight, and
   an inner-product operator configured to perform inner-product operation between the combined weight and the post-delay received signals, and generate the beamforming output.

7. The ultrasound imaging apparatus according to either of claim 4 and claim 5, wherein,
   the peripheral information combiner comprises,

plural inner-product operators configured to perform inner-product operation between the adaptive weights as to the plural points obtained by the peripheral information operator and the post-delay received signals respectively, and generate pre-synthesis beamforming outputs respectively for the plural adaptive weights, and

an output combiner configured to combine by adding the pre-synthesis beamforming outputs as to the plural adaptive weights, and generate the beamforming output that is used for generating the image data.

8. The ultrasound imaging apparatus according to any one of claims 1 to 7, further comprising,
the active channel setter configured to set active channels sequentially to the plural elements, at the positions being different in time series, and transfer to the delay unit, the received signals of the elements included in the active channels, wherein,
the peripheral information combiner combines the information items that the peripheral information operator obtains from the received signals in one of the active channels, and generates a final beamforming output that is used by the image processor.

9. The ultrasound imaging apparatus according to claim 6, further comprising,
the active channel setter configured to set active channels sequentially to the plural elements, at the positions being different in time series, and transfer to the delay unit, the received signals of the elements included in the active channels, wherein,
the peripheral information combiner sums the pre-synthesis beamforming outputs respectively as to the plural adaptive weights that the peripheral information operator obtains from the received signals in one of the active channels, and generates a final beamforming output that is used by the image processor, with respect to each of the active channels.

10. The ultrasound imaging apparatus according to claim 7, comprising a storing unit being arranged between the beamformer and the adder, the storing unit being configured to store the pre-synthesis beamforming outputs generated by the beamformer, as to each of the plural adaptive weights.

11. The ultrasound imaging apparatus according to claim 10, further comprising,
the active channel setter configured to set active channels sequentially to the plural elements, at the positions being different in time series, and transfer to the delay unit, the received signals of the elements included in the active channels, wherein,
the beamformer generates the pre-synthesis beamforming outputs respectively as to the plural adaptive weights, for each of the active channels, and
the adder acquires from the storing unit and the beamformer, the pre-synthesis beamforming outputs generated for the different active channels, and sums the beamforming outputs, so as to generate the beamforming output.

12. The ultrasound imaging apparatus according to claim 10, wherein,
the plural elements correspond to a two-dimensional array arranged two-dimensionally, and the peripheral information operator generates the pre-synthesis beamforming outputs as to the plural points being set in the two-dimensional direction, and the storing unit stores the pre-synthesis beamforming outputs.

13. The ultrasound imaging apparatus according to claim 5, comprising,
an adjuster configured to adjust at least either one of the number of the steering vectors and directions of the steering vectors.

14. The ultrasound imaging apparatus according to claim 13, further comprising,
a memory configured to store in advance a distribution of spread angles of the steering vectors directed to the plural points, in association with the position of the receive focus within the image that is generated by the image processor, wherein,
the adjuster obtains the adaptive weights, by using the steering vectors with the spread angles being read from the memory in association with the position of the receive focus.

15. The ultrasound imaging apparatus according to claim 13, further comprising,
a monitor for displaying an image, and an operating portion, wherein,
at least either of the monitor and the operating portion is provided with an accepting portion for accepting from an operator, a setting of the steering vectors in the adjuster.

FIG.1(a)

FIG.1(b)

# FIG.2

# FIG.3

# FIG.4

$$y(n) = \mathbf{w}^H(n)\mathbf{x}(n)$$

FIG.5(a)

FIG.5(b)

FIG.5(c)

FIG.5(e)

FIG.5(d)

FIG.5(f)

# FIG.6

(r-2)$^{th}$ RASTER 1501

(r-1)$^{th}$ RASTER 1502

r$^{th}$ RASTER 1503

$\theta_1$  $\theta_3$  $\theta_2$

1  2  3

$y_2(r-2,n)$

$y_1(r-2, n)$  $\Sigma$  $y_3(r-2, n)$

1504  $y_{sum}(r-2, n)$

BEAMFORMING
OUTPUT OF
THE (r-2)$^{th}$ RASTER 1501

1  2  3

$y_2(r-1,n)$

$y_1(r-1, n)$  $\Sigma$  $y_3(r-1, n)$

1505  $y_{sum}(r-1, n)$

BEAMFORMING
OUTPUT OF
THE (r-1)$^{th}$ RASTER 1502

1  2  3

$y_2(r,n)$

$y_1(r, n)$  $\Sigma$  $y_3(r, n)$

1506  $y_{sum}(r, n)$

BEAMFORMING
OUTPUT OF
THE R$^{th}$ RASTER 1503

# FIG.7

# FIG.8

$$y(n) = \mathbf{w}^{H}(n)\mathbf{x}(n)$$

# FIG.9

FIG.10(a)  FIG.10(b)  FIG.10(c)

FIG.11

# FIG.12

## FIG. 13

BEAMFORMING
OUTPUT OF
THE (r-2)^th RASTER 1501

BEAMFORMING
OUTPUT OF
THE (r-1)^th RASTER 1502

BEAMFORMING
OUTPUT OF
THE R^th RASTER 1503

# FIG.14

## FIG.15

BEAMFORMING OUTPUT
OF THE (r-1)$^{th}$ RASTER

# FIG.16

## FIG.17

(r-2)th RASTER 1501
1701
1500

(r-1)th RASTER 1502
1702
1500
1703

rth RASTER 1503
1704
1500

$\theta_1$  $\theta_3$
$\theta_2$

1  2  3

1  2  3

1  2  3

$\Sigma$

1901    500

BEAMFORMING OUTPUT
OF THE (r-1)th RASTER

# FIG.18

OUTPUT
ADJUSTER    *command*
503

900-(m+1)

[ 1,Q ]

800    900-m

[ 2,Q ]

FROM
BEAMFORMER
AND ADDER
307

[ 1,q ]

$y_{[1,Q]}(r,n)$

$y_{[2,Q]}(r,n)$

[P,Q]

[ 2,q ]

TO
BEAMFORMING
OUTPUT
COMBINER 500

$y_{[P,Q]}(r,n)$

902

[ 1,1 ]

900-
(m+1)

$y_{[1,q]}(r,n)$

$y_{[2,q]}(r,n)$

[P,q]

[ 2,1 ]

$y_{[P,q]}(r,n)$

900-m

900-1

$y_{[1,1]}(r,n)$

[P,1]

$y_{[2,1]}(r,n)$

$y_{[P,1]}(r,n)$

# FIG.19

FIG.20(a)

FIG.20(b)

FIG.20(c)

FIG.20(d)

FIG.20(e)

FIG.20(f)

FIG.20(g)

## FIG.21

FIG.22(a)

IMAGING AREA

2204
2204

2205
(SHALLOW PART)

2205 (SHALLOW
EDGE PART)

RASTER
SCANNING
DIRECTION

2205 (NEAR
SEND-FOCUS
POSITION)

2210

RASTER

2205 (DEEP
EDGE PART)

STEERING
ANGLE 2211

2204

2205
(DEEP PART)

2204

FIG.22(b)

B-MODE IMAGE

INTENSITY
LOWERING
AT SHALLOW PART

INTENSITY
LOWERING
AT EDGE IN
AZIMUTH
DIRECTION

2210

SUBSTANTIAL
IMAGING
AREA

INTENSITY
LOWERING
AT DEEP PART

FIG.22(c)

STEERING ANGLE
AT SHALLOW PART

STEERING ANGLE
AT EDGE PART

STEERING ANGLE
NEAR THE FOCUS

STEERING ANGLE
AT DEEP PART

2204        2205

FIG.22(d)

FIG.23(a)

SIGNAL STRENGTH OR INTENSITY

230

POSITION [cm]

FIG.23(b)

231 STEERING ANGLE DISTRIBUTION OBTAINED FROM THE DISTRIBUTION OF FIG.23(a)

SPREAD ANGLE

DIFFERENCE

POSITION [cm]

232 PREDETERMINED STEERING ANGLE DISTRIBUTION

FIG.24

103

1304

1303

110

1301

1302

# FIG.25

2300  2301  2302

2303  2304  2305

NO WAVEFRONT
DISTORTION EXISTS

WAVEFRONT
DISTORTION EXISTS

FIG.26(a)

FIG.26(b)

FIG.27

## INTERNATIONAL SEARCH REPORT

| International application No. |
| --- |
| PCT/JP2013/051305 |

A.  CLASSIFICATION OF SUBJECT MATTER
*A61B8/00(2006.01)i*

According to International Patent Classification (IPC) or to both national classification and IPC

B.  FIELDS SEARCHED

Minimum documentation searched (classification system followed by classification symbols)
A61B8/00

Documentation searched other than minimum documentation to the extent that such documents are included in the fields searched
Jitsuyo Shinan Koho          1922-1996    Jitsuyo Shinan Toroku Koho    1996-2013
Kokai Jitsuyo Shinan Koho    1971-2013    Toroku Jitsuyo Shinan Koho    1994-2013

Electronic data base consulted during the international search (name of data base and, where practicable, search terms used)

C.  DOCUMENTS CONSIDERED TO BE RELEVANT

| Category* | Citation of document, with indication, where appropriate, of the relevant passages | Relevant to claim No. |
| --- | --- | --- |
| X | JP 2011-45708 A  (Toshiba Corp.), 10 March 2011 (10.03.2011), entire text; all drawings & WO 2011/013713 A1      & CN 102365054 A | 1,2 |
| A | JP 2008-212492 A  (Hitachi Medical Corp.), 18 September 2008 (18.09.2008), entire text; all drawings & US 2010/0030081 A1      & WO 2008/108115 A1 | 1-15 |
| A | JP 2010-82371 A  (Canon Inc.), 15 April 2010 (15.04.2010), entire text; all drawings (Family: none) | 1-15 |

☒ Further documents are listed in the continuation of Box C.          ☐ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 06 February, 2013 (06.02.13) | 19 February, 2013 (19.02.13) |

| Name and mailing address of the ISA/ | Authorized officer |
| --- | --- |
| Japanese Patent Office | |
| Facsimile No. | Telephone No. |

Form PCT/ISA/210 (second sheet) (July 2009)

**INTERNATIONAL SEARCH REPORT**

International application No.

PCT/JP2013/051305

C (Continuation). DOCUMENTS CONSIDERED TO BE RELEVANT

| Category* | Citation of document, with indication, where appropriate, of the relevant passages | Relevant to claim No. |
|---|---|---|
| A | JP 2004-49926 A (Medison Co., Ltd.), 19 February 2004 (19.02.2004), entire text; all drawings & US 2004/0019277 A1 & KR 10-2004-0009256 A | 1-15 |
| A | JP 2003-225237 A (Hitachi Medical Corp.), 12 August 2003 (12.08.2003), entire text; all drawings & US 2003/0149362 A1 | 1-15 |
| A | JP 2003-79623 A (Medison Co., Ltd.), 18 March 2003 (18.03.2003), entire text; all drawings & US 2003/0045794 A1 & KR 10-2003-0021287 A | 1-15 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2010082371 A
- JP 2010063875 A
- JP 5249088 A
- JP 2002336249 A
- JP 7303640 A

### Non-patent literature cited in the description

- **I. K. HOLFORT et al.** Adaptive receive and transmit apodization for synthetic aperture ultrasound imaging. *Proc. IEEE Ultrason. Symp.,* 2009, 1-4
- **J. CAPON.** High-resolution frequency wavenumber spectrum analysis. *Proc. IEEE,* August 1969, vol. 57, 1408-1418
- **F. VIGNON et al.** Capon beamforming in medical ultrasound imaging with focused beams, IEEE Trans. Ultrason. *Ferroelectr. Freq. Control,* 2008, vol. 55 (3), 619-628
- **J. F. SYNNEVAG et al.** Benefits of Minimum-Variance Beamforming in Medical Ultrasound Imaging. *IEEE Trans. Ultrason, Ferroelectr. Freq. Control,* 2009, vol. 56 (9), 1868-1879
- **I. K. HOLFORT et al.** Broadband minimum variance beamforming for ultrasound imaging. *IEEE Trans. Ultrason. Ferroelectr. Freq. Control.,* 2009, vol. 56 (2), 314-325
- **Z. WANG et al.** Time-delay- and time-reversal-based robust capon beamformers for ultrasound imaging. *IEEE Trans. Med. Imag.,* 2005, vol. 24 (10), 1308-1322